# EUROPEAN PATENT APPLICATION

(11) **EP 1 182 255 A1**
(43) Date of publication of application: **27.02.2002**
(21) Application number: 00202867.8
(22) Date of filing: 16.08.2000
(51) Int. Cl.: C12N 15/12, A61K 48/00, C07K 14/47, C07K 14/705, C07K 16/18

(54) **Genes involved in immune related responses observed with asthma**

(71) Applicant: Universiteit Utrecht, 3584 CX Utrecht (NL)
(72) Inventor: Groot, Pieter, Cornelis, 2596 VK Den Haag (NL); Van Oosterhout, Antonius, Josephus Maria, 3573 PJ Utrecht (NL); Van Bergenhenegouwen, Bram Jeroen, 3581 EJ Utrecht (NL)
(74) Representative: Prins, Adrianus Willem

(57) **Abstract**

Asthma is one of the most common chronic diseases (155 million people worldwide) and is rapidly increasing (20-50% per decade), particularly in children (currently 10% in The Netherlands). Asthma impairs the quality of life and is a major cause of absence from school and work. Asthma, if not treated properly, can be life threatening. The invention provides a nucleic acid library comprising genes or functional fragments thereof said genes are essentially capable of initiation and/or progression and/ or suppression and/or repression of an immune response.

### Annex to the application documents - subsequently filed sequences listing

SEQUENCE LISTING

<110> Universiteit Utrecht

<120> Genes involved in immune related responses observed
      with asthma

<130> P53837EP00

<140> 00202867.8
<141> 2000-08-16

<160> 14

<170> PatentIn Ver. 2.1

<210> 1
<211> 4
<212> PRT
<213> Artificial Sequence

<220>
<223> Description of Artificial Sequence: caspase consensus site

<220>
<221> SITE
<222> (1)..(4)

<400> 1
Val Glu Val Asp
  1

<210> 2
<211> 4
<212> PRT
<213> Artificial Sequence

<220>
<223> Description of Artificial Sequence: internalization sequence

<220>
<221> SITE
<222> (1)..(4)

<400> 2
Asn Pro Thr Tyr
  1

<210> 3
<211> 20
<212> DNA

<213> Artificial Sequence

<220>
<223> Description of Artificial Sequence: sense primer

<220>
<221> misc_feature
<222> (1)..(20)

<400> 3
gccttcggac agcatttaca                                                    20

<210> 4
<211> 20
<212> DNA
<213> Artificial Sequence

<220>
<223> Description of Artificial Sequence: anti-sense primer

<220>
<221> misc_feature
<222> (1)..(20)

<400> 4
tgcgttgtcc aggtgataag                                                    20

<210> 5
<211> 24
<212> DNA
<213> Artificial Sequence

<220>
<223> Description of Artificial Sequence: sense primer

<220>
<221> misc_feature
<222> (1)..(24)

<400> 5
ggttgaggag cggaatggaa gagc                                               24

<210> 6
<211> 20
<212> DNA
<213> Artificial Sequence

<220>
<223> Description of Artificial Sequence: anti-sense primer

<220>
<221> misc_feature
<222> (1)..(20)

<400> 6
attgcccacg gcgctatcca                                                    20

<210> 7
<211> 25
<212> DNA
<213> Artificial Sequence

<220>
<223> Description of Artificial Sequence: sense primer

<220>
<221> misc_feature
<222> (1)..(25)

<400> 7
attagtcaca tttgacagcg ctgcc                                              25

<210> 8
<211> 24
<212> DNA
<213> Artificial Sequence

<220>
<223> Description of Artificial Sequence: anti-sense primer

<220>
<221> misc_feature
<222> (1)..(24)

<400> 8
tgggagacgc tgccacttgt agat                                               24

<210> 9
<211> 235
<212> PRT
<213> Mus musculus

<220>
<221> SITE
<222> (1)..(235)
<223> /note="IgE receptor beta chain"

<400> 9
Met Asp Thr Glu Asn Arg Ser Arg Ala Asp Leu Ala Leu Pro Asn Pro
  1               5                  10                  15

Gln Glu Ser Ser Ser Ala Pro Asp Ile Glu Leu Leu Glu Ala Ser Pro
            20                  25                  30

Ala Lys Ala Ala Pro Pro Lys Gln Thr Trp Arg Thr Phe Leu Lys Lys
        35                  40                  45

Glu Leu Glu Phe Leu Gly Ala Thr Gln Ile Leu Val Gly Leu Ile Cys
    50                  55                  60

Leu Cys Phe Gly Thr Ile Val Cys Ser Val Leu Tyr Val Ser Asp Phe
65                  70                  75                  80

Asp Glu Glu Val Leu Leu Leu Tyr Lys Leu Gly Tyr Pro Phe Trp Gly
                85                  90                  95

Ala Val Leu Phe Val Leu Ser Gly Phe Leu Ser Ile Ile Ser Glu Arg
            100                 105                 110

Lys Asn Thr Leu Tyr Leu Val Arg Gly Ser Leu Gly Ala Asn Ile Val
        115                 120                 125

Ser Ser Ile Ala Ala Gly Thr Gly Ile Ala Met Leu Ile Leu Asn Leu
    130                 135                 140

Thr Asn Asn Phe Ala Tyr Met Asn Asn Cys Lys Asn Val Thr Glu Asp
145                 150                 155                 160

Asp Gly Cys Phe Val Ala Ser Phe Thr Thr Glu Leu Val Leu Met Met
                165                 170                 175

Leu Phe Leu Thr Ile Leu Ala Phe Cys Ser Ala Val Leu Phe Thr Ile
            180                 185                 190

Tyr Arg Ile Gly Gln Glu Leu Glu Ser Lys Lys Val Pro Asp Asp Arg
        195                 200                 205

Leu Tyr Glu Glu Leu Asn Val Tyr Ser Pro Ile Tyr Ser Glu Leu Glu
    210                 215                 220

Asp Lys Gly Glu Thr Ser Ser Pro Val Asp Ser
225                 230                 235

<210> 10
<211> 244
<212> PRT
<213> Homo sapiens

<220>
<221> SITE
<222> (1)..(244)
<223> /note="IgE receptor beta chain"

<400> 10

Met Asp Thr Glu Ser Asn Arg Arg Ala Asn Leu Ala Leu Pro Gln Glu
1               5                   10                  15

Pro Ser Ser Val Pro Ala Phe Glu Val Leu Glu Ile Ser Pro Gln Glu
            20                  25                  30

Val Ser Ser Gly Arg Leu Leu Lys Ser Ala Ser Ser Pro Pro Leu His
        35                  40                  45

Thr Trp Leu Thr Val Leu Lys Lys Glu Gln Glu Phe Leu Gly Val Thr
    50                  55                  60

Gln Ile Leu Thr Ala Met Ile Cys Leu Cys Phe Gly Thr Val Val Cys
65                  70                  75                  80

Ser Val Leu Asp Ile Ser His Ile Glu Gly Asp Ile Phe Ser Ser Phe
                85                  90                  95

Lys Ala Gly Tyr Pro Phe Trp Gly Ala Ile Phe Phe Ser Ile Ser Gly
            100                 105                 110

Met Leu Ser Ile Ile Ser Glu Arg Arg Asn Ala Thr Tyr Leu Val Arg
        115                 120                 125

Gly Ser Leu Gly Ala Asn Thr Ala Ser Ser Ile Ala Gly Gly Thr Gly
    130                 135                 140

Ile Thr Ile Leu Ile Ile Asn Leu Lys Lys Ser Leu Ala Tyr Ile His
145                 150                 155                 160

Ile His Ser Cys Gln Lys Phe Phe Glu Thr Lys Cys Phe Met Ala Ser
                165                 170                 175

Phe Ser Thr Glu Ile Val Val Met Met Leu Phe Leu Thr Ile Leu Gly
            180                 185                 190

Leu Gly Ser Ala Val Ser Leu Thr Ile Cys Gly Ala Gly Glu Glu Leu
        195                 200                 205

Lys Gly Asn Lys Val Pro Glu Asp Arg Val Tyr Glu Glu Leu Asn Ile
    210                 215                 220

Tyr Ser Ala Thr Tyr Ser Glu Leu Glu Asp Pro Gly Glu Met Ser Pro
225                 230                 235                 240

Pro Ile Asp Leu

<210> 11
<211> 263
<212> PRT
<213> Mus musculus

<220>

<221> SITE
<222> (1)..(263)
<223> /note="LR8"

<400> 11
Met Val Gln Ser Thr Val Thr Val Asn Gly Val Lys Val Ala Ser Thr
1               5                   10                  15

His Pro Gln Ser Ala His Ile Ser Ile His Ile His Gln Lys Ser Ala
            20                  25                  30

Leu Glu Gln Leu Leu Gly Ala Val Gly Ser Leu Lys Lys Phe Leu Ser
        35                  40                  45

Trp Pro Gln Ala Arg Val His Tyr Gly Gln Leu Ser Leu Gly Val Thr
    50                  55                  60

Gln Ile Leu Leu Gly Leu Val Ser Cys Ala Leu Gly Val Cys Leu Tyr
65                  70                  75                  80

Phe Gly Pro Trp Thr Glu Leu Cys Ala Phe Gly Cys Ala Phe Trp Ser
                85                  90                  95

Gly Ser Val Ala Ile Leu Ala Gly Val Gly Thr Ile Val His Glu Lys
            100                 105                 110

Arg Gln Gly Lys Leu Ser Gly Gln Val Ser Cys Leu Leu Leu Leu Ala
        115                 120                 125

Cys Ile Ala Thr Ala Ala Ala Ala Thr Val Leu Gly Val Asn Ser Leu
    130                 135                 140

Ile Arg Gln Thr Ser Val Pro Tyr Tyr Val Glu Ile Phe Ser Thr Cys
145                 150                 155                 160

Asn Pro Leu Gln Ser Ser Met Asp Pro Gly Tyr Gly Thr Val Arg Tyr
                165                 170                 175

Ser Asp Asp Ser Asp Trp Lys Thr Glu Arg Cys Arg Glu Tyr Leu Asn
            180                 185                 190

Met Met Met Asn Leu Phe Leu Ala Phe Cys Ile Met Leu Thr Val Val
        195                 200                 205

Cys Ile Leu Glu Ile Val Val Ser Val Ala Ser Leu Gly Leu Ser Leu
    210                 215                 220

Arg Ser Met Tyr Gly Arg Ser Ser Gln Ala Leu Asn Glu Glu Glu Ser
225                 230                 235                 240

Glu Arg Lys Leu Leu Asp Gly His Pro Ala Pro Ala Ser Pro Ala Lys
                245                 250                 255

Glu Lys Ile Ser Ala Ile Leu
            260

<210> 12
<211> 270
<212> PRT
<213> Homo sapiens

<220>
<221> SITE
<222> (1)..(270)
<223> /note="LR8"

<400> 12
Met Thr Gln Asn Thr Val Ile Val Asn Gly Val Ala Met Ala Ser Arg
1               5                   10                  15

His Ala Gln Pro Thr His Val Asn Val His Ile His Gln Glu Ser Ala
            20                  25                  30

Leu Thr Gln Leu Leu Lys Ala Gly Gly Ser Leu Lys Lys Phe Leu Phe
        35                  40                  45

His Pro Gly Asp Thr Val Ser Ser Thr Ala Arg Ile Gly Tyr Glu Gln
    50                  55                  60

Leu Ala Leu Gly Val Thr Gln Ile Leu Leu Gly Val Val Ser Cys Val
65                  70                  75                  80

Leu Gly Val Cys Leu Ser Leu Gly Pro Trp Thr Val Leu Arg Ala Ser
                85                  90                  95

Gly Cys Ala Phe Trp Ala Gly Ser Val Val Ile Ala Ala Gly Ala Gly
            100                 105                 110

Ala Ile Val His Glu Lys His Pro Gly Lys Leu Ala Gly Tyr Ile Ser
        115                 120                 125

Ser Leu Leu Thr Leu Ala Gly Phe Ala Thr Ala Met Ala Ala Val Val
    130                 135                 140

Leu Cys Val Asn Ser Phe Ile Trp Gln Thr Glu Pro Phe Leu Tyr Ile
145                 150                 155                 160

Asp Thr Val Cys Asp Arg Ser Asp Pro Val Phe Pro Thr Thr Gly Tyr
                165                 170                 175

Arg Trp Met Arg Arg Ser Gln Glu Asn Gln Trp Gln Lys Glu Glu Cys
            180                 185                 190

Arg Ala Tyr Met Gln Met Leu Arg Lys Leu Phe Thr Ala Ile Arg Ala
        195                 200                 205

Leu Phe Leu Ala Val Cys Val Leu Lys Val Ile Val Ser Leu Val Ser
    210                 215                 220

Leu Gly Val Gly Leu Arg Asn Leu Cys Gly Gln Ser Ser Gln Pro Leu

225                 230                 235                 240

Asn Glu Glu Gly Ser Glu Lys Arg Leu Leu Gly Glu Asn Ser Val Pro
                245                 250                 255

Pro Ser Pro Ser Arg Glu Gln Thr Ser Thr Ala Ile Val Leu
            260                 265                 270

<210> 13
<211> 291
<212> DNA
<213> Artificial Sequence

<220>
<223> Description of Artificial Sequence: sequence R1-OS-B1-E5

<220>
<221> misc_feature
<222> (1)..(291)

<400> 13
gatcaaatgt attctttgtt acctcagggg cttcaggggc taggtgacaa ctccccaggg 60

ctagaaaggt aagcccatct aagtgttaca ggagtgattt tttgcctgtg actttgagat 120

gcccaattag aaccagcctc tggcccacct ntcctgggga agcgacaatg agattgcctt 180

tatacttcac ctggtgccta ggcttgctct taggcatcca ttttcctggt gagactttct 240

tcatgtcaca gaagccagca aataggtgga gtcacatgaa ggntcatgat c          291

<210> 14
<211> 480
<212> DNA
<213> Artificial Sequence

<220>
<223> Description of Artificial Sequence: sequence OtS2-C5

<220>
<221> misc_feature
<222> (1)..(480)

<400> 14
agattgtttg agggataatg atttcaagtg tttagcatct gagcctgcct gtaggttgat 60

ttcatctatt ttttagtaaa caagcattct ttgttagtca acatatcaca tcaaatatgc 120

ggagcatttt aaaaagctac ttcatccttt ggcattcaca tcatctagtc aaacagggtt 180

tcaagtgcag acggncactt gcacatgtag cgtggcacat canataccgg tgtgtgatgt 240

gaanctcaca caggggtgtt atctgaagca taatggtggc ttgggtaata tacatataag 300

ctcaanggna ccncgaattt ccttggaaag aacatnaacc tgggaanctt ttggngnccc 360

ttacctgcct gnctaanaaa cttgaccccc caaagattcn ctantaaccg ncgccantgt 420

gctnacnttc tncagatatt cctacacttg ngggcgntnn annncctctn nangggccca 480

## Description

The invention relates to the field of immunology, gene therapy and medicine. Asthma is one of the most common chronic diseases (155 million people worldwide) and is rapidly increasing (20-50% per decade), particularly in children (currently 10% in The Netherlands). Asthma impairs the quality of life and is a major cause of absence from school and work. Asthma, if not treated properly, can be life threatening.

Allergic asthma can be characterized by reversible airway obstruction, elevated levels of IgE, chronic airway inflammation and airway hyperresponsiveness to bronchoconstrictive stimuli, airway tissue remodeling and mucus hypersecretion. The allergic inflammatory infiltrate in the airway tissue predominantly consists of eosinophils and CD4⁺ T-lymphocytes. It is now widely accepted that type 2 T-helper (Th2) lymphocytes which produce a limited set of cytokines including interleukin-3 (IL3), IL4, IL5, IL9, IL10 and IL13 play an important role in the initiation and progression of allergic asthma [Corrigan and Kay (1992). *Immunology Today*. *13,* 501-507; Romagnani, S. (2000) *J Allergy Clin Immunol 105*, 399-408]. Chronic asthma appears to be driven and maintained by persistence of a subset of chronically activated memory T-cells (lymphocytes). Besides T-lymphocytes many other inflammatory cell-types are involved in the pathophysiology of allergic asthma such as eosinophils, mast-cells, B-lymphocytes, dendritic cells, macrophages and monocytes as well as resident airway cells such as epithelial cells and smooth muscle cells. Moreover, sensory neurons of which the cell bodies are located in the dorsal root ganglia play an important role in airway inflammation, hyperresponsiveness and cough.

Currently used pharmacological therapies in allergic asthma only provide temporal symptomatic relief. A more fundamental treatment aimed at antigen-specific T-lymphocytes and antigen-presenting cells is desirable since these cell-types play a crucial role in the initiation and progression of allergic asthma. Furthermore, T-lymphocytes may be the only cells that have the potential to induce long-term relieve of symptoms. Current therapy for moderate to severe asthma essentially involves multiple classes of molecules: anti-inflammatory glucocorticoids, bronchodilator drugs, and mast-cell inhibitors. The current preferred method is to treat the chronic phase of asthmatic symptoms, as manifested by airway hyperresponsiveness and eosinophilic inflammation, with glucocorticoids to reduce the inflammatory component and hyperresponsiveness (Barnes , 1990; Schleimer, 1990). These drugs are not very selective, targeting non-inflammatory cells as well as inflammatory cells and often have moderate to serious side effects after chronic treatment, especially in children. Furthermore, a subgroup (10%) of asthma patients become relatively resistant to glucocorticoid therapy and increasingly become dependent upon non-glucocorticoid treatment. In addition, there is a strong need for so-called "add-on" therapies to limit the use of high doses of glucocorticoids and the associated side-effects. Hence, there is a strong need for a safer, more selective and more efficacious therapeutic which displays a long-term clinical benefit to asthma patients.

The invention provides a nucleic acid library comprising genes or functional fragments, derivatives or analogues thereof essentially capable of modulating an immune response observed with airway hyper-responsiveness and/or bronchoalveolar manifestations of asthma. Modulation herein can refer to up-regulation or down-regulation of an immune response, for example by activation and/or suppression of gene(s) which are essentially capable of initiation and/or progression and/or suppression and/or repression of an immune response and/or symptoms of said immune response. Modulation herein can also refer, for example to positive (i.e up-regulation) or negative (i.e down-regulation) regulation of gene transcription, and to the modulation of the gene and gene product. Methods for modulating the expression of genes and gene products are known. The definition 'functional fragment thereof means that a particular subject sequence may vary from the reference sequence by one or more substitutions, deletions, or additions, the net effect of which does not result in an adverse functional dissimilarity between the reference and the subject sequence. An analogue is a compound having functional equivalence or being related to a molecule in question.

The invention provides a nucleic acid library comprising nucleic acid or functional fragments, derivatives or analogues thereof comprising at least one gene as listed in table 1, 2 or 3, genes which play an important role in all immune system related disorders such as all allergic diseases (asthma, rhinitis, atopic dermatitis, urticaria) and auto-immune diseases (i.e multiple sclerosis). The invention provides a nucleic acid library comprising such genes or fragments thereof said genes essentially capable of modulating an immune response observed with airway hyperresponsiveness and/or bronchoalveolar manifestations of asthma wherein said immune response is up-regulated and/or down-regulated. An immune response herein refers to the physiological response(s) stemming from activation of the immune system by antigens, including immunity to pathogenic organisms and auto-immunity to self-antigens, allergies, inflammatory response and graft rejection. An immune response herein further applies to all immune system related disorders. Usually the antigenic invader comprises a protein or protein attached moiety. The invention further provides a library comprising genes or functional fragments derivatives or analogue thereof said genes essentially capable of initiation and/or progression (i.e up-regulation) and/or suppression and/or repression (down-regulation) of an immune response wherein said immune response are airway hyperresponsiveness and/or broncheoalveolar manifestations of asthma. The invention provides a nucleic acid or functional fragments thereof selected from those listed in table 1, 2 or 3, capable of initiation and/or progression and/or suppression and/or repression of an immune response wherein said immune response is asthma. Methods of detecting nucleic acids capable of initiation and/or progression and/or suppression and/or repression of an immune response are known.

Changes in gene expression underlie most, if not all, pathophysiological processes. A variety of methods for detecting changes in gene expression in a healthy versus a diseased animal to detect nucleic acid for the formation of a library the subject of the invention are known. These procedures include, but are not limited to DNA-DNA or DNA-RNA hybridisation. The form of such quantitative methods may include, Southern or Northern analysis, dot/slot blot or other membrane based technologies; PCR technologies such as DNA Chip, Taqman®, NASBA, SDA, TMA, *in-situ*-hybridisation, protein bioassay or immunoassay techniques ELISA, IFA, proteomic and metabolomic technologies. These technologies are often found at the basis of my commercially available diagnostic kits often used for screening purposes.

The invention provides a nucleic acid library comprising genes or fragments thereof said genes essentially capable of modulating an immune response observed with airway hyperresponsiveness and/or bronchoalveolar manifestations of asthma wherein said genes comprises a nucleic acid essentially equivalent to a signature sequence as shown in table 1, 2 or 3. A signature sequence herein refers to a marker sequence and/or sequence or any other mode of identification of a sequence (i.e name). Nucleic acid sequence as used herein refers to an oligonucleotide, nucleotide or polynucleotide, and fragments or portions thereof, and to DNA or RNA of genomic or synthetic origin which may be single- or double-stranded, and represents the sense or antisense strand. The definition 'antisense' RNA is an RNA sequence which is complementary to a sequence of bases in the corresponding mRNA: complementary in the sense that each base (or majority of bases) in the antisense strand (read in the 5' to 3' sense) is capable of pairing with the corresponding base (G with C, A with U), in the mRNA sequence read in the 5' to 3' sense. The definition 'sense' RNA is an RNA sequence which is substantially homologous to at least part of the corresponding mRNA sequence. Preferably the nucleic acid is an 'immune response gene'. An immune response gene is any gene that determines the ability of lymphocytes to mount an immune response to specific antigens. The definition 'essentially equivalent' means that the subject signature sequence can vary from the reference sequence by one or more substitutions, deletions, or additions, the net effect of which will not result in a functional dissimilarity between the two sequences. It may be advantageous to produce nucleotide sequences, the subject of the invention or derivatives thereof possessing a substantially different codon usage. It is known by those skilled in the art that as a result of degeneracy of the genetic code, a multitude of gene sequences, some bearing minimal homology to the nucleotide sequences of any known and any naturally occurring genes may be produced. The invention includes each and every possible variation of the nucleotide sequences that could be made by selecting combinations based on possible codon choices.

The invention provides a library wherein said genes encode a regulatory molecule and/or co-stimulatory molecule and/or adhesion molecule and/or receptor molecule involved in modulating an immune response. The definition 'regulatory molecule' is an entity which assists the cell in 'sensing' it's environment. For example 'a regulatory molecule' can effect a immune response by modulating either positively or negatively gene transcription. The definition 'stimulatory molecule' is an entity which can activate an immune response. The definition 'adhesion molecules' is any pair of complementary molecules that bind specifically to one another to effect a positive or negative immune response. The molecule can be any entity which can bind to for example nucleic acid, proteinaceous substance or receptor etc., to effect a positive or negative immune response. The definition 'receptor' is an entity to which a ligand binds which triggers an immune response. The definition 'receptor molecule' could be for example a ligand (i.e any macromolecule) which binds to a receptor to effect an immune response. A ligand is a molecule that binds to a complementary site on a given structure. For example oxygen is a ligand for haemoglobin and a substrate of an enzyme molecule is a specific ligand of that molecule. The invention further provides a method for modulating an immune response of an individual comprising modulating a gene comprising a nucleic acid at least functionally equivalent to a nucleic acid identifiable by a signature sequence as shown in table 1, 2 or 3.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example Gob-5 (signature sequence R1-SO-R1-C11). Gob 5 is a cell-membrane protein belonging to the family of calcium-activated chloride channels and discovered in intestinal goblet cells in mice. Human CaCC1 and the identical CLCA1 are most likely the human homologs of murine gob-5. Gob-5 can have another function as a cell adhesion molecule. Northern blot analysis revealed that gob-5 is abundantly expressed in the stomach, small intestine, uterus and slightly expressed in the trachea of mice. *In-situ* hybridization demonstrated that gob-5 expression is located in the mucus-secreting cells of these three tissues. In humans, CaCC1/CLCA1 are also primarily expressed in the digestive tract. Gob-5 is expressed in lymph-nodes, lung tissue, bronchoalveolar lavage cells and bone-marrow from mice and is up-regulated in these tissues in the mouse asthma model. Mucus secreting goblet cells have never been described in lymph nodes or bone-marrow. In addition, Gob 5 is expressed in murine bone-marrow derived mast-cells and murine mast-cell lines. Gob-5 plays a role in secretory processes based on its function as a chloride channel. Chloride channels have been shown to be involved in mast-cell activation and degranulation since inhibition of these channels by non-selective broad spectrum chloride channel inhibitors inhibit IgE-mediated rat mast-cell degranulation *in-vitro*. Additionally a strong up-regulation of gob-5 in the dorsal root ganglia (DRG) in the mouse asthma model was observed. The expression of other members of the calcium-activated chloride channel gene family by PCR (table 2) was investigated. Murine homolog of human CaCC3 (EST AA726662) was identified and their expression was shown to be strongly upregulated in DRG of the mouse asthma model. In contrast, the expression of the murine homolog (m_CaCC or m_CLCA1) of human CLCA3 was strongly down-regulated in DRG from the mouse asthma model.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example LR8 (SEQ: ID: R1-OS-B1-D3). LR8 belongs to the family of the tetraspanin (4TM) superfamily. LR8 mRNA was not detectable by PCR in human smooth muscle cells, endothelial cells or epithelial cells. Murine LR8 mRNA expression in lymph nodes from mice was confirmed along with a down-regulation in a mouse asthma model. Bio-informatics analysis of the LR8 protein confirmed the presumed 4TM structure of the protein and revealed a striking homology with the beta chain of the high affinity IgE receptor (FcεRI). The tetraspanin superfamily has grown to nearly 20 known genes since its discovery in 1990. All encode cell-surface proteins that span the membrane four times, forming two extracellular loops. Many of these proteins have a flair for promiscuous associations with other molecules, including lineage-specific proteins, integrins, and other tetraspanins. In terms of function, they are involved in diverse processes such as cell activation and proliferation, adhesion and motility, differentiation, and cancer. These functions relate to their ability to act as "molecular facilitators," grouping specific cell-surface proteins and thus increasing the formation and stability of functional signaling complexes. LR8 is similar to CLAST1, a murine gene that is activated upon ligation of CD40 (Genbank: BAA83596). CD40 is predominantly expressed on so-called "antigen-presenting cells" and ligation of CD40 induces the expression of several molecules involved in the activation and regulation of T-lymphocytes (CD80; CD86; IL12). CD40 is an important maturation signal for dendritic cells. Immature dendritic cells take up antigen in peripheral tissues and migrate to secondary lymphoid tissues (draining lymph node) where they maturate and present antigen to lymphocytes. Several proteins are induced or down-regulated upon dendritic cell maturation. Many of the differentially activated genes appear to be involved in the modulation (regulation/activation) of T-lymphocytes (table 1, 2 or 3).

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example β-Amyloid-precursor like protein 2 (APLP2) (SvO2-1-B7). APLP2 is a highly conserved protein and is located on mouse chromosome 9. Moreover, in an experimental asthma model, airway hyper-responsiveness has been linked to a locus on chromosome 9, syntenic with human 11q24. APLP2 is a member of the Alzheimer precursor protein family including the Alzheimer peptide precursor (APP). These proteins all share three domains of similarity, interdispersed with completely divergent regions. APLP2 is a type-I integral membrane protein that contains a single membrane spanning domain with a large extracellular N-terminal domain and a short C-terminal cytoplasmic domain. APPL2 is ubiquitously expressed. Alternative splicing of APPL2 pre-mRNA generates at least four transcripts. Several functional domains have been identified in APLP2, including a DNA binding motif, an N-terminal cysteine rich domain exhibiting zinc, copper, and heparin binding activity, followed by a very acidic region and, depending on the isoform, the Kunitz protease inhibitor (KPI) domain. Interestingly, the KPI domain inhibits serine proteases like trypsin, plasmin, tryptase and chymase of which the latter two are released by activated mast-cells. Tryptase has been implicated in the development of airway hyperresponsiveness. Mast-cell mediator serotonin stimulates the release of APLP2 ectodomain (containing the KPI domain). Other functions that have been described for APLP2 are (i) an interaction with MHC class I, (ii) a role as adhesion molecule through interactions with extracellular matrix components, (iii) a role in epithelial wound healing and (iv) a potential role in the inhibition of platelet activation by the N-terminal cysteine-rich domain.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. For example the invention provides a method for the treatment of an immune response more particularly asthma and COPD comprising providing APLP2 or its KPI domain or by induction of APLP2 expression. APLP2 through the inhibition of the detrimental effects of mast-cell proteases, by repair of epithelial damage and by inhibition of platelet activation is capable of treating an immune related response. Furthermore, many allergens have been shown to have protease activities that appear to be crucial for allergic sensitization. By its KPI domain, APLP2 can inhibit the proteolytic activities of allergens and thereby prevent the initiation and progression of allergic responses. Another effect of the KPI domain of APLP2 is inhibition of the activation of protease-activated receptors (PARs) by serine proteases. PAR2 is involved in bronchorelaxation and protection against bronchoconstriction by stimulating the generation of prostaglandin E2 by airway epithelial cells. However, it was demonstrated that trypsin and a PAR2 ligand induced bronchoconstriction in guinea pigs *in vivo*, despite the induction of relaxation by these mediators in isolated trachea and bronchi. The bronchoconstriction appeared to be mediated by a neural mechanism since the bronchoconstriction was inhibited by the combination of NK1 and NK2 receptor antagonists. These data suggest that the PAR2 ligand activates sensory nerves. In agreement herewith, trypsin and mast-cell tryptase induced a wide-spread neurogenic inflammation initiated by activation of neuronal PAR2 receptors. Inhibition of tryptase and other serine proteases by APLP2 or its KPI domain can antagonize neurogenic inflammation and bronchoconstriction. Moreover, other PARs appear to be involved in inflammation. Activation of these receptors (PAR2) by serine proteases is sensitive to inhibition by APLP2 or its KPI domain. Analogous to intra-membrane cleavage of APP and Notch by aspartyl proteases (γ-secretase, presenilins). APLP2 can be cleaved by these aspartyl proteases since it is homologous to APP in the region (IATVIVI) where γ-secretase cleaves APP. This cleavage will lead to the generation of the extracellular part of APLP2 and an intracellular part of 57 amino acids, which may directly or indirectly modify the transcription of target genes. The APLP2 C57 peptide contains the "NPTY" sequence, which is present in many growth factor receptors and appears to be involved in cellular signaling. Interestingly, T-lymphocytes have been shown to express presenilin-1 and 2 at the cell-surface. Cleavage of APLP2 is involved in T-lymphocyte activation. Another, at present unidentified protease may cleave APLP2 in its transmembrane region and generate the release of an intracellular peptide containing the "NPTY" sequence.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. For example phosphotyrosine binding (PTB) domains have been identified in a large number of proteins. PTB domains play an important role in signal transduction by growth factor receptors. Several PTB proteins have been shown to bind to amyloid proteins through the "NPTY" motif like Fe65, Fe65-like, X11 and X11-like proteins, Shc and IRS-1. The interactions of APLP2 with Shc and IRS-1 is dependent on tyrosine phosphorylation whereas the interactions with Fe65 and X11 are not. The Fe65 adaptor protein interacts with the transcription factor CP2/LSF/LBP1. The "NPTY" motif, has been shown to be involved in binding to Shc, a Src homology 2 (SH2)-containing proto oncogene product implicated in activating Ras via association with Grb2 protein. Activation of the Ras pathway involves the MAPK signal transduction pathway which has been shown to be involved in the induction of many inflammatory genes. The Shc/Grb2/Sos complex is also involved in the activation of the Ras pathway in T-lymphocytes. It is unknown whether APLP2 or other proteins of this family with an "NPTY" domain are involved in T-cell activation and differentiation. Caspases can also cleave APP at the caspase consensus site "VEVD", leading to the generation of a C-terminal 31 amino acid peptide which contains the internalization sequence "NPTY". Since APLP2 contains both the caspase consensus site "VEVD" as well as the internalization sequence "NPTY", it is clear that APLP2 can also be cleaved by caspases leading to the generation of a C-terminal 31 amino acid peptide which is homologous to the peptide generated by APP cleavage. The APP C31 peptide has been demonstrated to initiate cell death. Apoptosis or cell-death is an important mechanism to limit immune and inflammatory reactions. On the other hand, cell-death may be unwanted i.e. death of airway epithelial cells may increase airway responsiveness. The invention provides a method for the treatment and/or prevention of an immune related response more particularly allergic asthma and related inflammatory diseases and COPD comprising modulating APLP2 or its KPI domain and/or by induction of APLP2 expression. Treatment by providing APLP2 or its KPI domain or induction of APLP2 expression is effective in the treatment of (1) the neurogenic component of inflammatory responses, (2) hyperalgesia during inflammatory responses, (3) cough due to airway inflammation and (4) bronchoconstriction induced by activation of sensory nerves. Cleavage of APLP2 by presenilins (γ-secretase) or other proteases or by caspase is involved in activation-induced cell-death in T-lymphocytes and is involved in the induction of peripheral tolerance.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma for example the invention provides a method for the treatment of immune responses comprising stimulating the cleavage of the intracellular domain of APLP2 by allosteric activation of proteases or by binding of APLP2 to its ligand together with an antigen-specific stimulation which will induce peripheral tolerance to the antigen. This treatment is effective for allergic asthma and other diseases mediated by T-lymphocytes such as auto-immunity and graft-rejection.

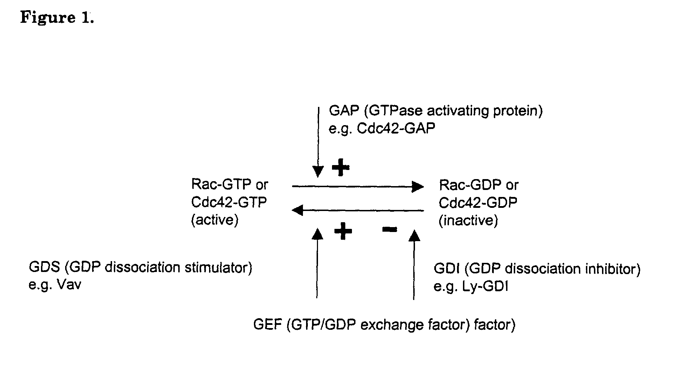

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example mouse GDP-dissociation inhibitor (Ly-GDI: signature sequence Sv-O2-1-D8). Ly-GDI was originally identified in lymphocytes and likewise called "lymphoid-specific GDI" (Ly-GDI). Independently, Ly-GDI gene was cloned from human and from mouse and it GDP-dissociation inhibitor D4 was designated. Mouse and human D4-GDI (Ly-GDI) share 89% amino acid sequence identity. Murine Ly-GDI is located on chromosome 6, the human homolog (Ly-GDI or D4-GDI) is located on chromosome 12p12.3. Northern blot analysis demonstrated that Ly-GDI was expressed abundantly in lung, and at lower levels in several other tissues. Another study using Northern blot analysis revealed that Ly-GDI is expressed as a 1.4-kb transcript only in hematopoietic tissues. Antibodies against Ly-GDI recognized a 27-kD protein on Western blots of B- and T-cell line lysates. It is now generally accepted that Ly-GDI is preferentially expressed in hematopotietic cells and can function as a GDP-dissociation inhibitor of Rho GTP binding proteins (Rac and Cdc42) but with less potency than the ubiquitously expressed RhoGDI. There are three subfamilies of small GTP-binding proteins, Ras, Rho and Rab. The present thinking is that Ras proteins are principally involved in signal transduction and cell proliferation, Rho proteins (Rac1, Rac2, TC10 and Cdc42) regulate cytoskeletal organization and Rab proteins are involved in the control of intracellular membrane traffic. The GTP-binding proteins are active only in the GTP-bound state. At least 2 classes of proteins tightly regulate cycling between the GTP-bound (active) and GDP-bound (inactive) states: GTPase-activating proteins (GAPs) and GDP/GTP exchange factors (GEF). GAPs inactivate small GTP-binding proteins by stimulating their low intrinsic GTPase activity to cause hydrolisis of GTP to GDP. GEFs are of two types including GDP dissociation stimulators (GDS, alternatively called guanine nucleotide releasing factors (GRF) and GDP-dissociation inhibitors (GDIs). The GDIs decrease the rate of GDP dissociation from Ras-like GTPases. It was found that Ly-GDI bound RhoA, and *in-vitro* inhibited GDP dissociation from RhoA. Stimulation of T lymphocytes with phorbol ester led to phosphorylation (activation) of Ly-GDI. It has been suggested that Ly-GDI may be involved in the regulation of hematopoietic-specific Rho-family GTPases because it is less potent than the ubiquitously expressed Rho-GDI. In T-lymphocytes, Rac and Cdc42 are important Rho-family GTPases involved in T-cell activation. Both Rac and Cdc42 are activated by Vav that has GDS activity (see figure 1). Rac and Cdc42 are involved in downstream signaling to the nucleus via the JNK pathway leading to the transcription factors AP1 (fos/jun) and NFAT (nuclear factor of activated T-cells). These transcription factors are involved in transcription of cytokines such as IL1, IL4, GM-CSF etc. Recently, it was demonstrated that Ly-GDI also interacts with the proto-oncogene Vav. Vav functions as a specific GDS for Rho, Rac and Cdc42 and is regulated by tyrosine phosphorylation in hematopoietic cells. Vav integrates signals from lymphocyte antigen receptors and co-stimulatory molecules to control development, differentiation and cell cycle. Interestingly, Vav knock-out mice have a defective IgE antibody production that can be attributed to compromised T cell help due to impaired IL-4 transcription. Ly-GDI knock-out mice have been generated and did not show striking abnormalities of lymphoid development or thymocyte selection. The mice also exhibited normal immune responses including lymphocyte proliferation, IL-2 production, cytotoxic T lymphocyte activity, antibody production, antigen processing and presentation, immune cell aggregation and migration, and protection against an intracellular protozoan. However, Ly-GDI-deficient mice exhibited deregulated T and B cell interactions after in vitro cultivation of mixed lymphocyte populations in concanavalin A (Con A) leading to overexpansion of B lymphocytes. Further studies revealed that Ly-GDI deficiency decreased IL-2 withdrawal-induced apoptosis of lymph node cells while dexamethasone- and T cell receptor-induced apoptosis remained intact. These data implicate the regulation of the Rho GTPase by Ly-GDI in lymphocyte survival and responsiveness, but suggest that these functions may be partially complemented by other Rho regulatory proteins when the Ly-GDI protein is deficient. Increased expression of GDP-dissociation inhibitor in the mouse asthma model in the lung-draining lymph nodes of "asthmatic" (OVA-challenged) compared to "healthy" (saline-challenged) mice was observed. A role for the GDP-dissociation inhibitor in the generation of Th2 immune responses is provided.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example a mouse fragment (signature sequence R1-SO-R1-A12) homologous to several mouse EST's and human (Cdc42-GAP) was identified. Human Cdc42 GTPase-activating (Cdc42-GAP) functions as a GAP for the Rho-family GTPase Cdc42 (See figure 1). Cdc42 can regulate the actin cytoskeleton through activation of Wiskott-Aldrich syndrome protein (WASP). Mutations in WASP lead to the Wiskott-Aldrich syndrome, a paediatric disorder characterized by actin cytoskeletal defects in heamatopoietic cells, leading clinically to thrombocytopenia, eczema and immunodeficiency. Recently, WASP-interacting protein (WIP) was shown to enhance the Vav-mediated activation of NF-AT/AP-1 gene transcription. Moreover, the interaction of WIP with WASP is necessary, but not sufficient for the ability of WIP to regulate NF-AT/AP-1 activity. Both Ly-GDI and Cdc42-GAP function in concert as inactivators of Cdc42. The invention provides a method for the treatment of immune responses more in particular allergic asthma and related allergic and Th2-mediated inflammatory diseases comprising providing blockade of Ly-GDI and/or Cdc42-GAP by selective antagonist(s) which inhibit T-helper lymphocyte type-2 (Th2) responses. The invention provides a method for the treatment of immune responses more in particular Th1-lymphocyte mediated diseases like auto-immune diseases comprising modulating Ly-GDI and/or Cdc42-GAP, more preferably inducing the expression of these proteins. Induction of the expression of these proteins induces T-helper lymphocyte type-2 responses and is therefore effective in the treatment of Th1-lymphocyte mediated diseases like auto-immune diseases.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example TIS11d/tristetraprolin homolog (signature sequence OtS2-A7). The human TIS11d protein is part of the TIS11 family of proteins also called tristetraprolin protein. These are basic proline-rich proteins and contain an unusual CCCH type of zinc finger structure. Tumor necrosis factor-α is a major mediator of both acute and chronic inflammatory responses in many diseases. In addition to its well-known role in acute septic shock, it has been implicated in the pathogenesis of chronic processes such as autoimmunity, graft-versus-host disease, rheumatoid arthritis, Crohn disease, and the cachexia accompanying cancer and AIDS. TIS11 interferes with TNF-α production by destabilizing its mRNA. This pathway represents a potential target for anti-TNF-α therapies. TIS11 deficiency also results in increased cellular production of granulocyte-macrophage colony-stimulating factor and increased stability of its mRNA, apparently secondary to decreased deadenylation. TIS11 is a physiologic regulator of GM-CSF mRNA deadenylation and stability. The invention provides a method for the treatment of an immune related response, comprising modulating expression, more preferably increased expression of TIS11d protein which inhibits the development of allergic asthma and related allergic and inflammatory diseases.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example many of the differentially activated genes as listed in table 1, 2 or 3 are involved in the regulation/activation of T-lymphocytes (T-lymphocyte activation molecules). Those up-regulated genes/proteins included terminal deoxynucleotidyl transferase (signature sequence: R1-SO-R1-E7), CsA-19 (signature sequence: ST-O1-B3), Pendulin (signature sequence: R1-SO-R1-E11), RA70 (signature sequence: STO1-D3), Ly-GDI (signature sequence SVO2-1-D8), Plastin-2 EST (signature sequence: SV02-1-C4), RNA Polymerase-II subunit EST (signature sequence: SV02-1-G3), Clathrin EST (signature sequence: SV02-1-D4), Cdc42-GAP (signature sequence: R1-SO-R1-A12). Those down-regulated genes/proteins were Stat-1 (signature sequence: R1-OS-B1-G3) IL2-R-gamma (signature sequence: OTS2-D9) IFN-γ-R (signature sequence: OTS2-A10).

The invention provides a method for modulating an immune response of an individual comprising modulating a gene comprising a nucleic acid at least functionally equivalent to a nucleic acid identifiable by a signature sequence as shown in table 1, 2 or 3, wherein said gene modulates a signal transduction cascade pertaining to an immune response. Method for modulating the expression of a nucleic acid are well known. In a preferred embodiment are nucleic acids as shown in table 1, 2 or 3 and functional equivalents whose products are capable of modulating genes of pathways central to immune response. 'Modulating' herein can also mean activation or suppression. More preferable is that the nucleic acid is involved in signal transduction cascades leading to suppression or activation of an immune responses. More preferable is that the nucleic acid encodes a proteinous substance (e.g a transcription factor) which may be involved in the activation or suppression of the Ras pathway in T-lymphocytes. Activation of the RAS pathway involves the MAP kinase (MAPK) signal transduction pathway which is involved in the induction of many immune related genes.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example LR8. LR8 is part of a multi-chain Fc receptor and is involved in the signal transduction by this Fc receptor upon ligand (immunoglobulin) binding. The invention provides a method for the treatment of an immune response comprising providing blockade of LR8. Blockade of LR8 prevents the activation of inflammatory cells through this Fc receptor. The invention provides a method for the treatment and/or prevention of an immune related response comprising modulating inhibition of aspartyl proteases such as presenilins (γ-secretase) involved in the cleavage of the intracellular 57 amino-acid part of APLP2 and blockade of the "NPTY" motif, which prevents activation of downstream signal transduction pathways including the Ras and MAPK pathway and associated changes in gene expression.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example Heat-shock protein 84 (Hsp84)(signature sequence: OTS2-C6). Hsp84 is a member of the Hsp90 family of heat-shock proteins. Hsp90 proteins are ubiquitous molecular chaperones with key roles in the folding, activation and assembly of a range of client proteins typically involved in signal transduction, cell cycle control or transcriptional regulation. Hsp90 has been shown to possess an inherent ATPase activity that is essential for the activation of authentic client proteins. Recently, Hsp90 and hsc70 (signature sequence: OTS2-H2) are both necessary and sufficient to activate hormone binding by the glucocorticoid receptor. A deficiency of Hsp90 or Hsp70 proteins may thus decrease the sensitivity of cells to the effects of glucocorticoids. In asthma, a gradual decrease in glucocorticoid sensitivity occurs. This decrease in glucocorticoid sensitivity can be mimicked by several cytokines e.g. IL-4. The invention provides a method for the treatment and/or prevention of an immune related response comprising modulating expression, more preferably increased expression of Hsp90 and/or Hsp70 proteins. This increases the sensitivity to the anti-inflammatory effects of glucocorticoids and is valuable in the treatment of asthma and other chronic inflammatory diseases.

Transcription factors are directed to the nucleus by their nuclear localization sequence (NLS) in a multistep process. The first step is to dock the NLS-containing protein to the nuclear pore and this is carried out by pendulin and Srp1. Pendulin (signature sequence R1-SO-R1-E11) contains an armadillo repeat region that is involved in NLS binding. Pendulin has been shown to be involved in the nuclear localization of lymphoid enhancer factor 1 (LEF-1) but not of the highly related T-cell factor 1 (TCF-1). Pendulin is the mouse homolog of human Rchl/Srp1α/importin-α. In contrast to a low-level of expression of mSrp 1 and pendulin in all tissues examined, mouse pendulin is highly expressed in spleen, thymus and heart. Pendulin may perform additional or unique functions in tissues that express high levels of this protein. Increased expression of pendulin in lymph nodes of the mouse asthma model was observed. The invention provides a method for treatment and/or prevention of an immune related response, more preferably asthma and related auto-immune and inflammatory diseases, comprising modulating expression of pendulin, more preferably increasing expression of pendulin.

The invention provides a method for modulating an immune response comprising modulating a gene(s) involved in signal transduction cascades leading to the production of cytokines and/or chemokines and/or growth factors pertaining to an immune response. Cytokines are primarily involved in signaling between cells of the immune system (e.g IL-4, IL-6, IL-8, IL-17 and Il-18). Chemokines are defined primarily as those compounds that draw cells and other factors to sites of injury in the body (e.g human GRO-β, Human IP-10). Growth factors promote cell division and proliferation of certain cell types (e.g human transforming growth factor β-1 etc).

The invention provides a method for modulating an immune response comprising modulating a gene, wherein said gene is involved in sensory nerve activation involved in an immune response. More preferably the immune response is an inflammatory response. Chloride channels appear to be involved in neuronal excitability. Dorsal root ganglia contain sensory nerve bodies that are involved in neurogenic inflammation which contributes to allergic inflammation and pain (inflammatory hyperalgesia). Interference with these chloride channels blockade of hCaCC1 (or gob-5) and/or hCaCC3 (or the murine homolog) by selective antagonists can limit neurogenic inflammation in asthma and other diseases with a neurogenic inflammatory component. Furthermore, cough, which is a prominent symptom of asthma, is believed to be a result of sensory nerve activation. The invention provides a method for the treatment of immune related responses comprising providing blockade of hCaCC1 (or gob-5) and/or hCaCC3 (or the murine homolog) by selective antagonists.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example blockade of hCaCC1 (or gob-5) by a selective antagonist inhibits mast-cell activation and can be used in diseases in which mast-cells play an important role such as all allergic diseases (rhinitis, atopic dermatitis, asthma, urticaria) and auto-immune diseases (i.e. multiple sclerosis). Blockade of hCaCC1 (or gob-5) and/or hCaCC3 inhibits the excitability of sensory neurons and thereby prevents or decreases (1) the neurogenic component of inflammatory responses, (2) hyperalgesia during inflammatory responses and (3) cough due to airway inflammation. Activation of receptors (PAR2) by serine proteases is sensitive to inhibition by APLP2 or its KPI domain and treatment with APLP2 or its KPI domain or induction of APLP2 expression is effective in the treatment of bronchoconstriction induced by activation of sensory nerves.

The invention provides a method for modulating an immune response comprising modulating a gene wherein said gene modulates a Th1 (by way of example but not limitation auto-immune diseases) and/or Th2 (by way of example but not limitation inflammatory diseases) mediated immune response. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance for example membrane C-type lectin like homolog (EST AA914211: signature sequence OtS1-B7). C-type (Ca²⁺-dependent) lectins represent an important recognition mechanism for oligosaccharides at cell surfaces, attached to circulating proteins and in the extra-cellular matrix. Binding of specific sugar structures by these lectins mediates biological events such as cell-cell adhesion, serum glycoprotein turnover and innate immune responses to potential pathogens. These proteins contain carbohydrate-recognition domains (CRDs) that mediate sugar binding. C-type lectins also contain a Ca²⁺ binding site. C-type lectins have been demonstrated to be present in antigen-presenting cells such as macrophages and dendritic cells. Interestingly, alveolar macrophages have been demonstrated to phagocytose allergens via an undefined C-type lectin leading to the induction of iNOS and subsequent generation of NO by alveolar macrophages. The NO generated by these macrophages may drive T-cell differentiation into the Th2 pathway by inhibition of Th1 responses. The invention provides a method for the treatment and/or prevention of an immune related response comprising providing the targeting of an antigen to this C-type lectin. This induces a Th2 dominated immune response and is effective in the treatment of Th1 mediated diseases such as auto-immune diseases.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example a protein inhibitor of neuronal nitric oxide synthase (mPIN) (signature sequence R1-OS-B1-B1). Nitric oxide (NO) can be produced by several nitric oxide synthase enzymes (nNOS, iNOS and eNOS). Murine PIN is a cytoplasmic protein and is a selective inhibitor of neuronal nitric oxide synthase (nNOS). The human homolog appears to be dynein light chain 1 (hdlc1). NO has been implicated in several diseases including asthma and other inflammatory diseases. Interestingly, nNOS is located on chromosome 12q that has been linked to asthma. The invention shows a down-regulation of mPIN mRNA in lymph nodes of a mouse asthma model. NO negatively regulates type-1 T-helper lymphocyte (Th1) development. Likewise, NO may tip the balance between Th1 and Th2 cells in favor of Th2 responses. The invention provides a method for the treatment and/or prevention of an immune related response, more particular Th2-mediated immune responses such as allergy and asthma comprising modulating PIN expression, more preferably decreasing expression which leads to increased NO release and facilitation of Th2-mediated immune responses such as allergy and asthma. The invention provides a method for the treatment and/or prevention of an immune related response, comprising blockade of PIN activity which is beneficial in Th1 mediated diseases such as auto-immunity by increasing regulatory Th2 cells. Treatment with PIN is beneficial in Th2 mediated responses such as asthma and allergy by increasing regulatory Th1 cells. Besides a role of PIN in the regulation T-cells, it plays a role in airway hyper-responsiveness. Neuronal NOS but not iNOS nor eNOS has been demonstrated to be crucial for baseline- and antigen-induced airway hyperresponsiveness in mice. Expression of nNOS but not eNOS nor iNOS in airway epithelial cells of our mouse model of allergic asthma is demonstrated. The invention provides a method for the treatment and/or prevention of an immune related response, comprising modulating nNOS and PIN, more preferably up-regulating nNOS in airway epithelial cells and down-regulating PIN. Up-regulation of nNOS in airway epithelial cells and a down-regulation of PIN can strongly potentiate the production of NO or its metabolites. The invention provides a method for the treatment and/or prevention of an immune related response, comprising modulating expression of PIN, more preferably increasing expression of PIN which inhibits NO production by nNOS and inhibits airway hyperresponsiveness in asthma and related respiratory diseases associated with hyperresponsiveness such as COPD.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma for example cathepsin B (signature sequence: OtS2-F2). Exogenous antigens are processed by lysosomal proteases within antigen-presenting cells to create antigenic peptides which are loaded into MHC class II molecules and expressed on the cell-surface to CD4⁺ T-lymphocytes. Enzymes such as aspartate proteases (e.g. cathepsin D and E) and cysteine proteases (e.g. cathepsin B, L and S) are proposed to be involved in this process. Interestingly, cathepsin B appears to be involved in the generation of Th2 dominated immune responses to ovalbumin and to a Leishmania infection in BALB/c mice. The invention provides a method for the treatment and/or prevention of an immune related response, comprising providing inhibition of the activity of cathepsin B by inhibitors. This inhibits allergic asthma and related allergic and Th2-mediated inflammatory responses.

Furthermore the invention provides a method for the treatment and/or prevention of an immune related response, comprising providing targeting of antigen to LR8 which will induce a Th2 dominated immune response and is effective in the treatment of Th1 mediated diseases such as auto-immune diseases. The invention provides a method for the treatment and/or prevention of an immune related response, comprising modulating Ly-GDI and/or Cdc42-GAP, more preferably inducing the expression of these proteins. Modulating Ly-GDI and/or Cdc42-GAP, or inducing the expression of these proteins induces T-helper lymphocyte type-2 responses and is effective in the treatment of Th1-lymphocyte mediated diseases like auto-immune diseases.

The invention provides a nucleic acid library comprising nucleic acid or functional fragments, derivatives or analogues thereof comprising genes as listed in table 1, 2 or 3 which are implicated in oxidative stress responses and/or programmed cell death (PCD) (i.e cellular apothosis). The invention provides a method for treatment of an immune response wherein said nucleic acid is involved in the generation of anti-oxidants or free radicals. An 'antioxidant' or free radical scavenger is an enzyme that prevents build up of reactive oxygen species (ROS) in cells. In general anti-oxidants prevent tissue damage by oxidative stress. Free 'radical generator' is a enzyme that is involved in the generation of ROS.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma for example clusterin/Apolipoproteine J/sulphated glycoprotein 2 (signature sequence OtS2-B12). Clusterin is a 75-80 kDa disulphide-linked heterodimeric secreted glycoprotein. It is encoded by a single gene and the translated product is internally cleaved to produce its α and β subunits prior to secretion from the cell. It is ubiquitously expressed. There is extensive evidence of a correlation between clusterin expression and diseases e.g. Alzheimer, glioma's or pathological stress. Many functions have been ascribed to clusterin such as controlling cell-cell and cell-substratum interactions; regulating apoptosis; transporting lipids; regulating complement and a general chaperone/heat-shock protein function.

The invention provides a method for the treatment and/or prevention of an immune related response, comprising modulating clusterin, more preferably increasing the expression of clusterin, which will inhibit allergic asthma and related allergic and inflammatory diseases.

Moreover, anti-oxidants may inhibit the expression of genes regulated by the "redox status" within inflammatory cells, such as the ras pathway. Oxidative stress also appears to be involved in the activation of the CD4-associated protein tyrosine kinase p56^{lck}. P56^{lck} is an important protein in the activation of CD4⁺ T-lymphocytes. Oxidative stress is increased in patients with asthma and chronic obstructive pulmonary disease (COPD) and it is possible that reactive oxygen species contribute to its pathophysiology. Likewise, antioxidants might be of use in the therapy of these respiratory diseases. Oxidative stress has also been shown to regulate the cellular glucocorticoid responsiveness. A decreased sensitivity to glucocorticoids has been observed in patients with allergic asthma leading to treatment with either high-doses of glucocorticoids or inappropriate treatment. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention provides various anti-oxidant proteins down-regulated upon OVA-challenge in the mouse asthma model e.g Selenoprotein P (signature sequence: R1-OS-B1-H1), Gluthation-S-transferase mu2 (signature sequence: OtS2-E6), Ferritine (signature sequence: R1-OS-B1-O5), Anti-oxidant protein 2 (signature sequence: OtS2-A6).

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma for example selenium. Selenium is an essential trace element that is incorporated as selenocysteine into the primary structure of selenoproteins. There are at least 10 animal selenoproteins. Animal studies have demonstrated a role for selenium in oxidant defense, thyroid hormone metabolism, and defense against viral infections. Selenoproteins presumably mediate these biologic effects. Most of the human selenoproteins are members of the gluthatione peroxidase or iodothyronine deiodinase families. Selenoprotein P (SEPP1) is not a member of these families. It is an extracellular glycoprotein that is present in several isoforms and is the only selenoprotein known to contain multiple selenocysteine residues. It is a heparin-binding protein that appears to be associated with endothelial cells and has been implicated as an oxidant defense in the extracellular space. There is evidence that several isoforms of the protein exist, likely products of the same gene. Human selenoprotein has been mapped to chromosome 5q31. Interestingly, many studies have demonstrated a linkage between chromosome 5q and allergy, asthma and airway hyperreactivity. There is considerable evidence that oxidative stress is increased in patients with chronic obstructive pulmonary disease (COPD) and that reactive oxigen species contribute to its pathophysiology. Likewise, it has been postulated that antioxidants might be of use in the therapy of COPD. Selenoprotein P may be useful as a therapeutic protein in diseases that are associated with increased oxidative stress such as COPD, asthma and other inflammatory diseases. It was observed that mRNA levels of selenoprotein P are decreased in lymph node tissue of a mouse asthma model. Selenium and selenoproteins have been shown to play a role in the function of granulocytes and lymphocytes. The invention provides a method for the treatment and/or prevention of an immune related response, comprising modulating selenoprotein P.

The invention provides a method for modulating an immune related response, comprising modulating the generation of anti-oxidants or free radicals. Treatment with anti-oxidant proteins (e.g by inhalation) or induction of the expression of these proteins and/or suppression of free radical generators in airway tissue can be used to treat allergic inflammation or related inflammatory diseases or diseases associated with increased oxidative stress such as asthma and COPD. Treatment with anti-oxidant proteins or induction of the expression of these proteins in airway tissue together with glucocorticoid treatment can limit the dose of glucocorticoids required for a therapeutic effect in patients with allergic asthma and other chronic inflammatory diseases associated with glucocorticoid insensitivity.

The invention provides a substance such as a proteinaceous substance capable of modulating a gene comprising a nucleic acid at least functionally table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma for example cytochrome P-450 naphtalene hydroxylase (CYP2F2) enzymes (signature sequence R1-OS-B1-A1). CYP2F2 are a superfamily of more than 160 known members that play a major role in the metabolism of numerous physiological substrates and a wide array of xenobiotics including drugs, chemical carcinogens, insecticides, petroleum products, and other environmental pollutants. Oxidative metabolism catalyzed by cytochrome P450s can result in detoxification. In some instances it results in metabolic activation of a chemical to cytotoxic and/or carcinogenic forms. Although the liver is the primary organ for drug metabolism, extrahepatic tissues such as lung, kidney and intestine, also play an important role in detoxification or biotransformation of xenobiotics. Each tissue has a unique P450 isozyme distribution and regulatory mechanism for cytochrome P450 gene expression. Currently, the members of the CYP2F gene subfamily that are selectively expressed in lung tissues consist of human CYP2F1 and mouse CYP2F2 and CYP2F3. Human CYP2F1 bioactivates 3-methylindole, while mouse CYP2F2 bioactivates naphtalene. Mouse CYP2F3 catalyzes the dehydrogenation of 3-methylindole but not its hydroxylation. Murine CYP2F2 is expressed in lung tissue as well as in liver. In the lung, it plays an important role in the metabolic activation of substrates that cause lung injury. CYP2F2 is involved in the hydroxylation of naphtalene and it specifically catalyses the production of a very reactive and potentially toxic intermediate, the 2R, 2S arene oxide, that is associated with necrosis of unciliated bronchiolar epithelial cells or CLARA cells in lung. Several P450 enzymes with epoxygenase activity have also been shown to be involved in the metabolism of arachidonic acid into biologically active eicosanoids. Based on the bioactivation of naphtalene, we anticipate that CYP2F enzymes also displays epoxygenase activity. The epoxygenase pathway leads to the formation of four regio-isomeric epoxyeicosatrienoic acids (EETs): 14,15-EET, 11,12-EET, 8,9-EET and 5,6-EET. From these epoxides, other lipid mediators can be generated such as 14,15-DHET, 11,12-DHET, 8,9-DHET, 5,6-DHET and 5,6-epoxy prostaglandin E1. Some of these epoxides have been shown to induce vasorelaxation. 5,6-EET and 11,12-EET have also been shown to modulate tracheal chloride-channel activity and induce airway smooth muscle relaxation. Epoxides generated through CYP2F may therefore protect against excessive bronchoconstriction and may be involved in airway hyperreactivity in asthma and other respiratory diseases. Epoxygenase metabolites have also been shown to have anti-inflammatory activities such as inhibition of leukocyte adhesion to the vascular wall and inhibition of IκB kinase thereby preventing the activation of NF-κB. Cytochrome P-450 naphtalene hydroxylase (CYP2F2). A strong (>10-fold) down-regulation of cytochrome P450 (CYP2F2) mRNA in a mouse asthma model in the lymph nodes of "asthmatic" (OVA-challenged) compared to "healthy" (saline-challenged) mice was observed. The invention provides a method for the treatment and/or prevention of an immune related response, comprising modulating the expression of CYP2F, more preferably increasing expression of CYP2F in airway tissue and/or by preventing its down-regulation. This inhibits airway hyperresponsiveness and excessive bronchoconstriction and can be used to treat allergic asthma and other respiratory diseases associated with hyperresponsiveness such as COPD. The invention provides a method for the treatment and/or prevention of an immune related response, comprising providing local treatment (inhalation) with CYP2F metabolites of arachidonic acid, in particular 11,12-EET, which inhibits airway inflammation for treatment of allergic asthma and other respiratory inflammatory diseases such as COPD. The invention provides for a method of treatment and/or prevention of an immune related response, comprising modulating the enzymatic activity of CYF2F, more preferably stimulating the enzymatic activity of CYF2F by an allosteric stimulator which increases the generation of epoxides and likewise inhibits airway hyperresponsiveness and airway inflammation. Stimulation of the enzymatic activity of CYF2F by an allosteric stimulator is effective in the treatment of allergic asthma and other respiratory diseases such as COPD.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example four families of structurally related heat-shock proteins are distinguished based on their molecular weights: Hsp90, Hsp70, Hsp60 and small Hsp's. By definition, Hsp expression is elevated in cells undergoing stress, such as those in damaged or inflamed tissue. Conditions as diverse as a rise in temperature, hypoxia, irradiation, infection and exposure to toxic chemicals can all result in increased Hsp expression. Heat-shock cognate protein (Hsc)73 is a constitutively expressed member of the Hsp70 family. Hsc73 is expressed in the cytosol but is also present in lysosomes. Hsc73 plays a role in binding and protecting peptides from extensive degradation and facilitating the kinetics of peptide transfer to MHC class II molecules. Hsc73 is also present in dendritic cell-derived exosomes which have been shown to elicit potent T-cell dependent immune responses in mice. Moreover, a receptor for Hsp70 proteins is present on the surface of macrophages and dendritic cells and Hsp70 can induce macrophages to activate T-cells independently of antigen. Thus, Hsc73 appears to be involved in antigen-presentation and T-cell activation. Administration of antigen or antigenic peptides together with Hsp70 proteins has been shown to generate CD8⁺ T-lymphocyte responses when administered to laboratory animals. Moreover, Hsp70 is involved in cross-priming of CD8⁺ cells by APC upon antigen processing. Recently, Hsp70 has also been shown to be involved in the induction of regulatory T-cells. Hsc73 (signature sequence: OtS2-H2) may also be involved in the induction of inducible nitric oxide synthase (iNOS) by LPS or cytokines via an effect on p38 mitogen-activated protein (MAP) kinase. In agreement herewith, the selective hsc73 inhibitor deoxyspergualin inhibits the induction of iNOS by cytokine- or endotoxin-activated macrophages. NO has been shown to inhibit the generation of Th1 lymphocytes thereby tipping the balance towards Th2 immune responses. In airway epithelial cells, Hsp70 has been shown to have potent anti-inflammatory effects by stabilization of IκBα through preventing the activation of IκB kinase leading to inhibition of NF-κB activation and down-stream gene transcription. In airway epithelial cells, increased Hsp70 expression suppressed cytokine-induced expression of pro-inflammatory cytokines IL8 and TNFα.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against Hsc73 . This inhibits the generation of NO by APC's and thereby limits a Th2 dominated immune response by increasing Th1 immunity. This treatment is effective in the treatment of allergic asthma and related allergic and inflammatory responses.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example, the invention provides for a method of treatment and/or prevention of an immune related response, more particularly allergic inflammation or related inflammatory diseases (e.g. COPD) comprising modulating, more preferably up-regulating the expression of Hsc73 leading to induction and/or elevation of the expression of Hsc73 protein in airway epithelial cells.

The invention provides a method for treatment of an immune response comprising providing an antagonist of antigen processing and presentation. 'Antagonist' herein refers to a molecule that bears sufficient structural similarity to a second molecule to compete with that molecule for binding sites on a third molecule, such as for example an antibody. An 'antibody' herein refers to a protein produced by lymphoid cells in response to foreign substances (antigens) and capable of coupling specifically with it's homologous antigen (the one that stimulated the immune response) or with substances that are chemically very similar to that antigen. Antibody herein refers to both polyclonal and monoclonal antibodies.

The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance by way of example the invention provides nucleic acids as listed in table 1, 2 or 3 which are involved in antigen processing and presentation MHC-II (signature sequence: StO1-B5), H2-Oa (MHC-II: signature sequence: SvO2-1-A4), EST: Clathrin (signature sequence: SvO2-1-D4), Aspartyl aminopeptidase (signature sequence: StO1-c1), Cathepsin B (signature sequence: OtS2-F2), Breast heat shock 73 protein (signature sequence: OtS2-H2), EST: C-type lectin (signature sequence: OtS1-B7), Ubiquitin-specific protease (signature sequence: R1-OSB1-A2), Ubiquitin/60s (signature sequence: SVO2-1-C12) and Lysozyme M (OtS2-B1). Antigen-presenting cells play an important role in the differentiation of CD4⁺ and CD8⁺ T-lymphocytes into particular subsets (Type-1, Type-2, Type-3 or regulatory types) and are important for the generation of either a detrimental or a beneficial immune response to antigens.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example Phospholipase Cγ2 (PLCγ2). (signature sequence: SvO2-1-A8). PLCγ2 unlike PLCγ1 which is expressed in many cell-types, PLCγ2 is only expressed in hematopoietic cells (e.g. B-lymphocytes, NK-cells, platelets, granulocytes, monocytes/macrophages and mast cells). PLCγ2 is a cell signaling molecule with many regulatory domains e.g. SH2, SH3, pH domains. It catalyzes the hydrolysis of phosphatidyl-inositol 4,5-biphosphate to yield the second messengers, IP3 and DAG. PLCγ2 has been shown to be involved in production of reactive oxygen intermediates by neutrophils. In addition to PLCγ1, PLCγ2 is activated upon triggering of mast-cells via Fcε RI. The promotor region of PLCγ2 has Sp1, NF1, AP2, SRE, EBF and CACCC box consensus sites. In B-cells, mRNA expression of PLCγ2 is enhanced by serum, TPA, retinoic acid and 5-azacytidine. The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against PLCγ2 or a proteinaceous substance comprising PLCγ2 is effective in the treatment of allergic asthma and related allergic and inflammatory diseases.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example APLP2 C31 (signature sequence: SvO2-1-B7) peptide is involved in cell death (apoptosis). Apoptosis or cell-death is an important mechanism to limit immune reactions. The cytoplasmic domain of APLP2 containing the "NPTY" motif is involved in T-lymphocyte activation upon phosphorylation of the tyrosine (Y) residue leading to Shc binding. The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against APLP2, more specifically the cytoplasmic domain of APLP2 containing the "NPTY" motif. This prevents the Ras-pathway of T-lymphocyte activation and inhibits an immune response and is effective in the treatment of allergic asthma and related allergic and inflammatory diseases. The invention provides for a method of treatment and/or prevention of an immune related response, comprising providing an antagonist(s) directed against "VEVD" and "NPTY" motif inhibits unwanted cell death mediated by this pathway and is effective in the treatment of allergic asthma and related allergic and inflammatory diseases. The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing inhibition of the generation of the C-terminal 31 amino acid APLP2 peptide by caspases and/or proteases encoded by the nucleic acid of table 1, 2 or 3 which inhibits unwanted cell death mediated by this pathway.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against hCaCC1 (or gob-5) (signature sequence: R1-SO-R1-C11) which inhibits mast-cell activation and can be used in the treatment of immune diseases in which mast-cells play an important role such as all allergic diseases (rhinitis, atopic dermatitis, asthma, urticaria) and auto-immune diseases (i.e. multiple sclerosis).

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1, 2 or 3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against Hsc73. This inhibits the generation of NO by APC's and thereby limits a Th2 dominated immune response by increasing Th1 immunity. This treatment is effective in the treatment of allergic asthma and related allergic and inflammatory responses.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1, 2 or 3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against LR8 which inhibits allergic asthma and related allergic and inflammatory diseases.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma. More preferably a method of treatment and/or prevention of an immune related response, more preferably allergic asthma and related allergic and inflammatory diseases, comprising providing an antagonist(s) directed against one or more up-regulated genes as listed in table 1, 2 or 3 or the corresponding proteinaceous substances.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against Ly-GDI (signature sequence: SVO2-1-D8) and/or Cdc42-GAP (signature sequence: R1-SO-R1-A12) which inhibits T-helper lymphocyte type-2 (Th2) responses and is effective in the treatment of allergic asthma and related allergic and Th2-mediated inflammatory diseases.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma. For example the invention provides for a method of treatment and/or prevention of an immune related response, comprising providing an antagonist(s) directed against C-type lectin (signature sequence: Ot-S2-B7) which inhibits antigen presentation and skewing towards a Th2 dominated immune response. This blockade is effective in the treatment of allergic asthma and related allergic and inflammatory diseases.

The invention provides a method for modulating an immune response wherein said gene modulates CD8⁺ T-lymphocyte responses. Also provided is a gene or gene product capable of inducing a specific regulatory CD4⁺ and/or CD8⁺ T-lymphocyte response that inhibits Th2 dominated allergic responses. The invention provides a method for modulating an immune response wherein said gene modulates CD4⁺ T-lymphocyte responses. The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma for example Ubiquitin-specific protease (UBP43)(signature sequence R1-OS-B1-A2). UBP43 belongs to a family of ubiquitin-specific proteases (UBP) and has a molecular mass of 43 kDa. Protein ubiquitination has been implicated in many important cellular events. The human homolog of this protein is ISG43. In wild-type adult mice, UBP43 is highly expressed in thymus and peritoneal macrophages. Furthermore, it is expressed in cell-lines of the monocytic lineage and its expression is regulated during cytokine-induced monocytic cell differentiation. Over expression of UBP43 has been shown to block cytokine-induced terminal differentiation of the monocytic cell-line M1. Down-regulation of UBP43 mRNA in lymph nodes of a mouse asthma model was observed. The invention provides for a method of treatment and/or prevention of an immune related response, comprising modulating the expression of UBP43, more preferably increasing the expression of UBP43 in APC's which prevents allergic asthma and related respiratory disease by increasing the generation of regulatory CD8⁺ T-lymphocytes. The proteasome is involved in the generation of MHC class-I peptides by proteases.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance. The invention further provides the use of said antagonist such as an antibody directed against a proteinaceous substance derived from at least a nucleic acid as shown table 1-3 for the production of a medicament for the treatment of an immune response observed with airway hyperresponsiveness and/or bronchoalveolar manifestations of asthma. For example the invention provides for a method of treatment and/or prevention of an immune related response, comprising providing inhibition of peptide loading into MHC class-I molecules by proteases encoded by the nucleic acid as outlined in table 1, 2 or 3, which inhibits the generation of CD8⁺ T-lymphocyte responses (i.e T-lymphocyte costimulation). Airway wall remodeling is an established pathological feature of asthma but its causes are not well understood. One cytokine of potential relevance is transforming growth factor-beta1 (TGF-beta 1). In patients with asthma, matrix-associated TGF-beta 1 is likely to be bound at least in part to decorin (signature sequence: R1-OS-B1-C5). This interaction may provide a reservoir of TGF-beta 1 that can be released in an active form in response to appropriate stimuli. Decorin is also a natural inhibitor of TGF-beta and has been shown to restore T-lymphocyte responses to mycobacteria. The invention provides for a method of treatment and/or prevention of an immune related response, comprising modulating the expression of decorin, preferably increasing the expression of decorin. Increased expression of decorin in airway tissue and/or treatment (inhalation) with decorin inhibits the effects on TGF-beta on airway tissue remodeling and is effective in the treatment of immune related responses.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance for example the invention provides for a method of treatment and/or prevention of an immune related response, comprising providing immunotherapy using Hsc73, alone or together with antigen/allergen. An allergen herein is defined as a substance inducing hypersensitivity. Immunotherapy using Hsc73, alone or together with antigen/allergen induces a specific regulatory CD4⁺ or CD8⁺ T-lymphocyte response that inhibits Th2 dominated allergic responses.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance for example the invention provides a method for modulating an immune response of an individual wherein said gene encodes a gene product capable of modulating an immune response. A gene product herein refers the mRNA and the polypeptide chain translated from an mRNA molecule, which in turn is transcribed from a gene; if the RNA transcript is not translated (e.g rRNA, tRNA) the RNA molecule represents the gene product. The gene product herein can refer to any proteinaceous substance. A proteinaceous substance can refer to any molecule comprising amino acid and/or peptide or protein.

The invention provides alleles of the polypeptide(s) encoded by nucleic acid sequences of this invention. As used herein, an 'allele' or 'allelic sequence' is an alternative form of the polypeptides described above. Alleles result from a mutation [eg. a change in the nucleic acid sequence, and generally produce altered mRNA or polypeptide whose structure or function may or may not be altered]. Any given polypeptide may have none, or more allelic forms. Common allelic changes that give rise to alleles are generally ascribed to natural deletions, additions or substitutions of amino acids. Each of these types of changes may occur alone, or in combination with the others, one or more times in a given sequence. Deliberate amino acid substitution may be made on the basis of similarity in polarity, charge, solubility, hydrophobicity, and/or the amphipathetic nature of the residues as long as the biological activity of the polypeptide is retained. Altered nucleic acid sequences of this invention include deletions, insertions, substitutions of different nucleotides resulting in the polynucleotides that encode the same or are functionally equivalent. A 'deletion' is defined as a change in either nucleotide or amino acid sequence in which one or more nucleotides or amino acid residues, respectively, are absent. An 'insertion' or 'addition' is that change in nucleotide or amino acid sequence which has resulted in the addition of one or more nucleotides or amino acid residues, respectively, as compared to the naturally occurring polypeptide(s). A 'substitution' results from the replacement of one or more nucleotides or amino acids by different nucleotides or amino acids, respectively. The invention includes variants of the polypeptide. A 'variant' of a polypeptide is defined as an amino acid sequence that is different by one or more amino acid 'substitutions'. A variant may have 'conservative' changes, wherein a substituted amino acid has similar structural or chemical properties eg replacement of leucine with isoleucine. More rarely a variant may have 'non-conservative' changes (eg replacement of a glycine with a tryptophan). Similar minor variations may also include amino acid deletions or insertions, or both. Guidance in determining which and how many amino acid residues may be substituted, inserted or deleted, without abolishing biological or immunological activity may be found using computer programs well known in the art, for example, DNAStar software.

The invention provides a method modulating an immune response wherein said immune response comprise airway hyperresponsiveness and/or bronchoalveolar manifestations of asthma.

The invention provides a method modulating an immune response wherein said gene is modulated by transducing a cell of said individual. Methods to transduce cells are known in the art. Target cells can be transduced with a nucleic acid delivery vehicle comprising at least one nucleic acid the subject of the invention. A 'gene delivery vehicle' herein is used as a term for a recombinant virus particle or the nucleic acid within such a particle, or the vector itself, wherein the vector comprises the nucleic acid to be delivered to the target cell(s) and is further provided with a means to enter said cell(s). This cell(s) can be used for drug screening and drug discovery.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance, for example the invention provides a substance capable of modulating a gene comprising a nucleic acid at least functionally equivalent to a nucleic acid identifiable by a signature sequence as shown in table 1, 2 or 3.

A substance herein refers to any material entity capable of modulating a gene the subject of the invention, for example an 'entity' can be a molecule wherein said molecule is a chemical compound. The substance can also be an 'antigen' a foreign invader comprising a protein or protein attached moiety. The substance can also be of proteinaceous origin comprising amino acid and/or peptide or protein.

The invention provides a medicament comprising a substance capable of modulating a gene(s) the subject of the invention. A preferred embodiment is a medicament which is a pharmaceutical. Suitable pharmaceutical compositions are known.

The invention provides the use of a substance for the production of a medicament for the treatment of an immune response observed with airway hyperresponsiveness and/or bronchoalveolar manifestations of asthma.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance, for example the invention provides the use of a proteinaceous substance derived from a nucleic acid at least functionally equivalent to a nucleic acid identifiable by a signature sequence as shown in table 1, 2 or 3 for the production of an antagonist against said substance. 'Antagonist' herein refers to a molecule that bears sufficient structural similarity to a second molecule to compete with that molecule for binding sites on a third molecule, for example an antibody.

The invention provides the use of a proteinaceous substance derived from a nucleic acid at least functionally equivalent to a nucleic acid identifiable by a signature sequence as shown in table 1, 2 or 3 for the production of an antagonist against said substance, wherein said antagonist is an antibody or functional equivalent thereof. An 'antibody' herein refers to a protein produced by cells in response to foreign substances (antigens) and capable of coupling specifically with it's homologous antigen (the one that stimulated the immune response) or with substances that are chemically very. similar to that antigen. Antibody herein refers to both polyclonal and monoclonal antibodies.

The invention provides for a method of treatment and/or prevention of an immune related response, comprising providing an antagonist(s) directed against a proteinaceous substance derived from a nucleic acid sequence at least functionally equivalent to a nucleic acid identifiable by a signature sequence as shown in table 1, 2 or 3. The invention provides a substance such as a proteinaceous substance capable of modulating a gene comprising a nucleic acid at least functionally equivalent to a nucleic acid identifiable by a signature sequence as shown in table 1-3 and use of said substance for the production of an antagonist against said substance for example the invention provides an antagonist directed against a proteinaceous substance derived from a nucleic acid at least functionally equivalent to a nucleic acid identifiable by a signature sequence as shown in table 1, 2 or 3. 'Functionally equivalent' herein means that the subject signature sequence can vary from the reference sequence by one or more substitutions, deletions, or additions, the net effect of which will not result in a functional dissimilarity between the two sequences.

The invention provides an antagonist comprising an antibody or functional equivalent thereof. An antibody or functional equivalent thereof can refer to synthetic molecules (i.e antibodies derived by chemical synthesis) and encompasses all molecules capable of coupling with proteinaceous substance(s) derived from nucleic acid of the invention. Proteinaceous substance herein can refer to an entity derived from said nucleic acids the subject of the invention capable of modulating an immune response.

The invention provides a medicament comprising an antagonist. The invention provides the use of an antagonist for the production of a medicament for the treatment of an immune response observed with airway hyperresponsiveness and/or bronchoalveolar manifestations of asthma.

The invention provides for use of an antagonist for the production of a medicament for the treatment of an immune response observed with airway hyperresponsiveness and/or bronchoalveolar manifestations of asthma.

The invention further provides a diagnostic kit for screening for an immune response comprising providing a nucleic acid the subject of the invention. Methods of screening are known in the art. These procedures include, but are not limited to DNA-DNA, DNA-RNA hybridisation. The form of such quantitative methods may include, Southern or Northern analysis, dot/slot blot or other membrane based technologies; PCR technologies such as DNA Chip, Taqman®, NASBA, SDA, TMA, *in-situ*-hybridisation, protein bioassay or immunoassay techniques ELISA, IFA, proteomic and metabolomic technologies.

### Examples

### Example 1: Development of murine model of allergic asthma

Due to the limitations of experimental studies in patients with allergic asthma a murine model with immunologic and pathophysiologic features reminiscent of allergic asthma was developed [Oosterhout AJ (1998): *Am J Respir Cell Mol Biol;* 19:826-35]. There are several advantages to using a murine model compared to using tissues obtained from asthma patients such as (i) availability of isolated tissues or cells (ii) genetic homogeneity, (iii) identical age, (iv) well-controlled environment (food, specified pathogens, climate), and (v) ability to do time-series experiments (i.e induction vs effector phase). In this model, Balb/c mice are sensitized with ovalbumin (OVA) and repeatedly challenged by inhalation of OVA aerosol. This model is characterized by the presence of OVA-specific IgE antibodies in serum, airway eosinophilia and non-specific hyperresponsiveness concomitant with the appearance of Th2-like cells in lung tissue and lung draining (thoracic) lymph nodes.

### Example 2: Representational Difference Analysis (RDA)

Representational Difference Analysis of cDNA's (RDA) was employed to identify novel key regulatory molecules involved in the initiation and/or progression and/or suppression and/or repression of asthma symptoms. RDA analysis was performed according to previously defined methods [Groot and van Oost (1998). *Nucleic Acids Res:* 26:4476-81][Welford *et al.,* (1998): Nucleic Acids Res 1998; 26:3059-65] [Geng et al., (1998): *Biotechniques* 25:434-8]. Gene expression between lung-draining lymph nodes (containing amongst others dendritic cells, macrophages, B- and T-lymphocytes, mast-cells) obtained from "healthy" control animals and those obtained from "asthmatic" mice that display airway manifestations of asthma such as airway hyperresponsiveness and bronchoalveolar eosinophilia were compared. Balb/c mice were intraperitoneally sensitized with ovalbumin and later on repeated challenged by inhalation of saline aerosol (control or "healthy" animals) or ovalbumin aerosol ("asthmatic"). Lymph nodes were isolated at 6 hours after the last challenge. Using RDA differentially expressed gene fragments were identified. Up-regulated genes are those that are expressed at higher levels in asthmatic tissue compared to healthy tissue. Vice versa, down-regulated genes are those that are expressed at lower levels in asthmatic tissue compared to healthy tissue. NCBI (National center of biotechnology information) BLAST searches with the differentially expressed gene fragments against publicly available databases revealed significant alignment with either known genes (human or mouse), with expressed sequence tags (EST's) or in some cases did not reveal a significant alignment or an incomplete alignment (unknown genes). The identified differentially expressed genes are listed in Table 1.

### Example 3: Microarray experiment

Detection of differentially expressed genes in "asthmatic" mice compared with "healthy" control animals was performed using representational differences analysis coupled to microarray hybridization methods as described previously [Welford *et al*., (1998). *Nucleic Acids Res:* 26:3059-65]. Unique differentially expressed genes (tethered nucleic acid: target) obtained from the RDA experiment (example 2) were amplified by PCR using M13 primers, precipitated and spotted (arrayed in duplicate) onto chemically-modified glass slides (Corning) using a robotic printing device. Messenger RNA obtained from both lymph nodes of "healthy" and from "asthmatic" mice was transcribed into double-stranded cDNA and amplicons were generated. Amplicons were subsequently fluorescently labeled with either cyanine 3 (Cy3-ULS) or cyanine 5 (Cy5-ULS) dyes (i.e one mRNA population (probe: free nucleic acid) was labeled with cyanine 3 (Cy3-ULS) and the other with cyanine 5 (Cy5-ULS)). The labeled probes (free nucleic acids) were then mixed and hybridized simultaneously to a microarray. The microarray was hybridized with both the Cy3 and Cy5 labeled probes in order to determine the expression ratio between both samples. After hybridization, the fluorescence pattern of each microarray was recorded for the Cy3 and Cy5 fluorescent dyes. Detailed statistical analyses were applied in order to determine the minimal significant ratio in each experiment. Clones that exhibited differential fluorescence were identified. In table 1, the expression ratio ("asthma" : "healthy") is given.

### Example 4: Virtual Northern Blot

Messenger RNA obtained from lymph nodes of "healthy" and "asthmatic" mice was transcribed into double-stranded cDNA and amplicons were generated. Using agarose gel electrophoresis, different amounts of amplicons were run and subsequently blotted onto Hybond filter membrane. Specific and individual gene fragments obtained by RDA from the lymph nodes of "healthy" and "asthmatic" mice were subcloned and subsequently amplified using M13 primers and fluorescently labeled (by random primer labeling). Labeled gene fragments were hybridized on the filter membrane containing the blotted amplicons and analyzed by a fluor-imager. After hybridization, based on the fluorescence intensity between amplicons obtained from "healthy" and "asthmatic" mice, an expression ratio ("asthma":"healthy") was determined (table 1).

### Example 5: By way of example one novel therapeutic target protein for the treatment of immune and/or inflammatory responses.

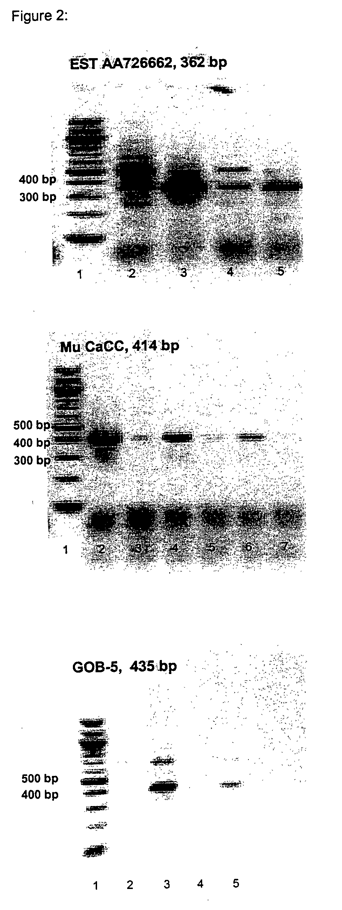

The mRNA expression of gob 5 has been examined by PCR using gene-specific primer pairs (sense primer: GCCTTCGGACAGCATTTACA; anti-sense primer TGCGTTGTCCAGGTGATAAG; product length 435 base-pairs). Gob 5 mRNA is present in lymph nodes, lung tissue, bronchoalveolar lavage cells and bone-marrow obtained from healthy BALB/c mice. In tissues obtained from "asthmatic" mice compared to tissues obtained from "healthy" mice, the expression of gob 5 mRNA is increased in lymph nodes (approximately 4 fold), bronchoalveolar lavage cells (>10 fold), and bone marrow cells (approximately 2 fold). Mucus secreting goblet cells have never been described in lymph nodes or bone-marrow. The expression of gob 5 in murine bone-marrow derived mast-cells and murine mast-cell lines is demonstrated (P815 and CFTL-12). Additionally, a strong up-regulation of gob 5 in the dorsal root ganglia (DRG) obtained from the mouse asthma model was observed (figure 2). The expression of other members of the calcium activated chloride channel family was determined by PCR (table 1, Table 2 and figure 2). We have identified a murine homolog of CaCC3 (EST AA726662) and we show that the expression is strongly upregulated (>16 fold) in DRG of the mouse asthma model compared to healthy mice (figure 2). In contrast, the expression of the murine homolog (m_CaCC or m_CLCA1) of human CLCA3 was strongly down-regulated in DRG from the mouse asthma model (figure 2).

### Example 6 By way of example one novel therapeutic target protein for the treatment of immune and/or inflammatory responses

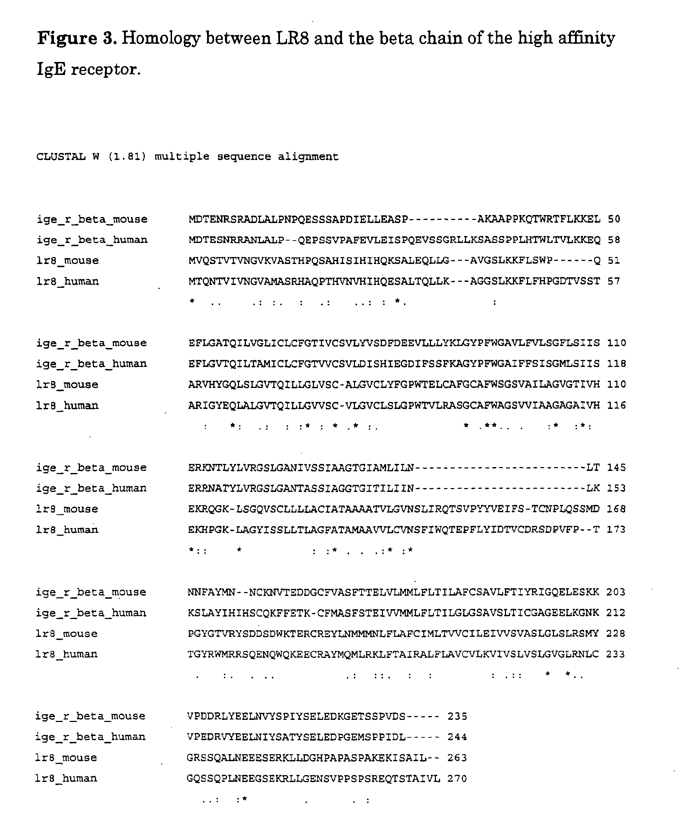

LR8/CLAST1 belongings to the family of the tetraspanin (4TM) superfamily and has been discovered in a subpopulation of human lung fibroblasts. LR8 mRNA was not detectable by PCR in human smooth muscle cells, endothelial cells or epithelial cells. A murine homolog of LR8 (Signature sequence R1-OS-B1-D3) showed gene (i.e mRNA) expression in lymph nodes from mice and a down-regulation in the mouse asthma model. Bio-informatics analysis of the LR8 protein confirmed the presumed 4TM structure of the protein and revealed a striking homology with the beta chain of the high affinity IgE receptor (Fcε RI) (Figure 3).

### Tables

**Table 1 :** Identification of differentially expressed genes in "asthmatic" mice compared with "healthy" control animals. **Array** ^{**1**}**:** Expression ratio (asthma:healthy) obtained by hybridization of a cDNA micro-array with fluorescently labeled amplicons (Cy5 versus Cy3) derived from "asthma" and "healthy" mice.
**Blot** ^{**2**}**:** Expression ratio (asthma:healthy) obtained by virtual northern blotting of amplicons and hybridization with fluorescently labeled specific, individual gene fragments.

**Table 2:** members of the calcium-activated chloride channel family.

**Table 3:** An example of some of the differentially expressed genes involved in the regulation/activation of T-lymphocytes from table 1.

**Table 1.**

| **a) Known genes up-regulated in "asthma" versus "healthy" mice.** | | | | |
|---|---|---|---|---|
| **Signature Sequence** | **Sequence/gene** | **Human homolog** | **Array** ¹ | **Blot** ² |
| R1-SO-R1-A11 | Igγ | IgGγ | 2.09 | 10 |
| StO1-A10 | Igε | Igε | 2.08 | |
| SvO2-1-C11 | Igµ | Igµ | | |
| StO1-A12 | IgG1 H chain | IgG1 H chain | 2.20 | |
| R1-SO-R1-B7 | Igκ | Igκ | 2.36 | 4 |
| R1-SO-R1-A7 | SLPI (secretory leukocyte protease inhibitor) | SLPI | 3.19 | 10 |
| R1-SO-R1-E7 | Tdt (terminal deoxynucleotidyl transferase) | Tdt | 3.65 | |
| StO1-B3 | CsA-19 | CsA-19 | 1.57 | |
| StO1-B5 | MHC-II (I^{A-d}) | MHC-II | 3.11 | |
| R1-SO-R1-C11 | Gob-5 (ca²⁺ activated Cl⁻channel) | CaCC1/CLCA1 | 1.88 | 2 |
| R1-SO-R1-E11 | Pendulin | Rch1/Srp1α/ Importin-α | 0.84 | 2 |
| R1-SO-R1-A12 | EST AA277412; AW910210; AI591665; AA980800 | CDC42-GAP (GTPase-activating protein) | 1.02 | 2 |
| StO1-C1 | Aspartyl aminopeptidase | Aspartyl aminopeptidase | 1.41 | |
| StO1-D3 | RA70 (mouse retinoic acid responsive gene) | SKAP-HOM (SKAP55 homolog) | 0.77 | |
| SvO2-1-B7 | APLP2 (amyloid β precursor-like protein) | APLP2 | | |
| SvO2-1-D8 | GDP-dissociation inhibitor (ly-GDI) | Ly-GDI | | |
| SvO2-1-C4 | Plastin-2 (PLS2) | L-Plastin | | |
| SvO2-1-C12 | Ubiquitin/60s | | | |
| SvO2-1-A4 | H2-Oa (MHC-II) | HLA-DNA | | |
| SvO2-1-G3 | EST AI327412; AA140026 | RNA Polymerase-II subunit (POLR2G) | | |
| SvO2-1-A8 | EST AW546508 | Phospholipase-C γ2 (PLCγ2) | | |
| SvO2-1-D4 | EST AW044803; AA823969; AA869959 | Clathrin (CLTCL2) | | |
| SvO2-1-D5 | EST BB000142 | Glutamyl-propyl-tRNA synthethase (EPRS) | | |

| **b) Expressed sequence tags (EST's) up-regulated in "asthma" versus "healthy" mice** | | | | |
|---|---|---|---|---|
| **Signature sequence** | **Sequence/gene** | **Human homolog** | | |
| SvO2-1-D10 | EST AI153476; AA537538 | | | |
| SvO2-1-A11 | EST AI451488 | AW173082 | | |
| SvO2-1-C8 | EST AA023597; AW476575 | | | |
| SvO2-1-E6 | EST AI587693; AA499481; AU080538 | | | |
| SvO2-1-F1 | EST C77954 | | | |

| **c) Known genes down-regulated in "asthma" versus "healthy" mice.** | | | | |
|---|---|---|---|---|
| **Signature sequence** | **Sequence/gene** | **Human homolog** | **Array** ^{**1**} | **Blot** ^{**2**} |
| R1-OS-B1-B1 | PIN (protein inhibitor of NnoS) | Dynein light chain | 1.44 | 0.7 |
| R1-OS-B1-A1 | CYP2F2 (cytochrome P450 naphthalene hydroxylase) | CYP2F1 | 0.35 | 0.1 |
| R1-OS-B1-B6 | IDH-α (NAD⁺ dependent isocitrate dehydrogenase) | NAD⁺ dependent isocitrate dehydrogenase | 0.71 | 0.5 |
| R1-OS-B1-G3 | Stat-1 | Stat-1 | 0.65 | 0.3 |
| R1-OS-B1-H1 | SEPP1 Selenoprotein P | SEPP1 | 0.52 | 0.5 |
| R1-OS-B1-C5 | Decorin | Decorin | 0.40 | 0.3 |
| OtS2-F2 | Cathepsin B | Cathepsin B | 0.56 | |
| OtS2-E6 | Gluthation-S-transferase mu 2 (Gstm2) | Gluthation-S-transferase | 0.40 | |
| OtS2-H2 | Breast heat shock 73 protein (Hsc73) | HSP 70 | 0.60 | |
| OtS2-B12 | Sulphated glycoprotein-2 isoform APOJ/Clu | Clusterin | 0.46 | |
| R1-OS-B1-D3 | LR8/CLAST1 | LR8 | 0.54 | 0.5 |
| R1-OS-B1-C1 | EST AW211263; AI194829; AI098607; W08910 | Mitochondrial trifunctional protein | 0.55 | 0.7 |
| R1-OS-B1-A2 | UBP43 (ubiquitin specific protein) | ISG43 | 0.80 | 0.5 |
| R1-OS-B1-D5 | Ferritine | Ferritine | 0.45 | 1.0 |
| OtS2-B4 | Unidentified mitochondrial gene | | 0.50 | |
| OtS2-A1 | Mitochondrial cyt-C oxidase subunit I | | 0.43 | |
| OtS2-C10 | Mitochondrial enoyl-CoA hydratase (rat) | Mitochondrial enoyl-CoA hydratase | 0.34 | |
| OtS2-A6 | AOP2 (antioxidant protein 2) | AOP2 | 0.45 | |
| OtS2-D9 | IL-2R-γ | IL-2R-γ | 0.51 | |
| OtS2-A7 | EST AA475628 | TIS11d (early response gene) /tristetraprolin | 1.12 | |
| OtS2-C6 | HSP (84 kd heat shock protein) | HSP 90 | 0.75 | |
| OtS2-A10 | IFNγR (interferon-γ receptor) | IFNγR | 0.32 | |
| OtS2-C11 | Ornithine decarboxylase (Odc) | Ornithine decarboxylase | 0.55 | |
| OtS1-C11 | Stearoyl-CoA desaturase 1 (SCD1) | Stearoyl-CoA desaturase | 0.38 | |
| OtS2-B10 | MUSLYSM4 (mouse lysozyme gene) | | 0.54 | |
| OtS2-D8 | Calnexin | Calnexin | 0.61 | |
| R1-OS-B1-D6 | Plunc | Plunc | 0.39 | |

| **d) Expressed sequence tags (EST's) down- regulated in "asthma" versus "healthy" mice.** | | | | |
|---|---|---|---|---|
| **Signature sequence** | **Sequence/gene** | **Human homolog** | **Array** ^{**1**} | **Blot** ^{**2**} |
| OtS2-D3 | EST AI451901; AW826053; AA712022, partially similar to mouse CR2 | | 0.74 | |
| OtS2-D2 | EST AA423205, similar to X57528 mouse retinoic acid receptor-alpha | | 0.87 | |
| OtS2-D10 | Similar but not identical to mouse CD59 (complement inhibitory protein) | | 0.53 | |
| OtS1-B7 | EST AA543877; AA914211 (similar but not identical to macrophage lectin-2) | Similar but not identical to membrane C-type lectin 2 | 0.43 | |
| R1-OS-B1-C3 | EST AA691014; AW321759 | | 0.84 | 0.5 |
| OtS2-G2 | Mouse JHL1 (AF165227) | | 0.58 | |
| R1-OS-B1-H6 | EST AI450028, AW548213; AA672579 | MUM2 (AF129332) | 0.83 | 0.25 |
| R1-OS-B-A3 | EST AA512682; AI314236 | | 0.65 | 0.7 |
| R1-OS-B1-C4 | EST AA396183 (similar to rat ROD1) | ROD1 | 0.66 | 0.5 |
| R1-OS-B1-A5 | EST AW490156 (similarity to dynein beta subunit) | EST AI358291; AI623698 | 1.02 | 0.3 |
| R1-OS-B1-B2 | EST AI835555 | | | 0.7 |
| OtS2-C1 | EST AA939676; AA125221; AA798681; AA869527 | | 0.77 | |
| OtS2-D7 | EST AU078971; AA178650; AA231343 | | 1.60 | |
| OtS2-B9 | EST AA792488; AA177706 | | 0.37 | |
| OtS2-A9 | EST AA273304; AA270364; AA671609 | AF143676 (multi-spanning nuclear envelope membrane protein) | 0.56 | |
| R1-OS-B1-C6 | EST AI874718; AA498063; AA615985 | | 0.81 | |
| OtS2-C3 | EST AI788596; AI892968; AA939676 | | 0.66 | |
| OtS2-B6 | EST AI528153; AA982059; AW488424 | | 0.67 | |
| OtS2-A12 | EST AA940560 (Rho-GAP domain) | AF217507 | 0.65 | |
| OtS2-B3 | EST AL022972 | AW958031 | 1.43 | |
| OtS2-A5 | EST AA433598; AL118320; AI507121 | | 1.08 | |
| OtS2-C4 | EST AW913417; AI647667 | | 0.68 | |

| **e) Genes down-regulated in "asthma" versus "healthy" mice.** | | | | |
|---|---|---|---|---|
| **Signature sequence** | **Sequence/gene** | **Human homolog** | **Array** ^{**1**} | **Blot** ^{**2**} |
| R1-OS-B1-E5 | See figure 4 for sequence | | 0.97 | 0.7 |
| OtS2-C5 | See figure 4 for sequence | | 0.35 | |

**Table 2:**

| members of the calcium-activated chloride channel family. | | |
|---|---|---|
| **Human gene/protein** | **Murine homolog** | **Signature sequence** |
| CaCC1 / CLCA1 | Gob-5 | R1-SO-R1-C11 |
| CaCC2 | EST W41083 | |
| CaCC3 / CaCL2 | EST AA726662 | |
| CLCA3 | CaCC / CLCA1 | |

**Table 3.**

| T-lymphocyte activation/regulation molecules: | | | |
|---|---|---|---|
| **Up-regulated genes/proteins** | **Signature sequence** | **Down-regulated genes/proteins** | **Signature sequence** |
| | | | |
| CsA-19 | St-O1-B3 | IL2-R-gamma | OTS2-D9 |
| Pendulin | R1-SO-R1-E11 | IFN-γ-R | OTS2-A10 |
| RA70 | StO1-D3 | Stat-1 | R1-OS-B1-G3 |
| Ly-GDI | SV02-1-D8 | | |
| Plastin-2 | SVO2-1-C4 | | |
| EST: RNA Polymerase-II subunit | SVO2-1-G3 | | |
| EST: Clathrin | SVO2-1-O4 | | |
| EST: Cdc42-GAP | R1-SO-R1-A12 | | |

### Figure legends

**Figure 1:** Effects of Ly-GDI and Cdc42-GAP on small GTP-binding proteins Rac and Cdc42
**Figure 2:** PCR products using cDNA obtained from dorsal root ganglia (DRG) isolated from "healthy" or "asthmatic" mice. PCR was carried out using conditions well known in the art using the gene-specific primer pairs for:
   a) EST AA726662 (Top)(sense primer: GGTTGAGGAGCGGAATGGAAGAGC; antisense primer: ATTGCCCACGGCGCTATCCA, product length 362 base pairs);
   b) m_CaCC (Middle)(sense primer: ATTAGTCACATTTGACAGCGCTGCC; antisense primer: TGGGAGACGCTGCCACTTGTAGAT, product length 414 base-pairs);
      and for
   c) gob 5 (Bottom)(sense primer: GCCTTCGGACAGCATTTACA; anti-sense primer TGCGTTGTCCAGGTGATAAG; product length 435 base-pairs).
   Lane 1 refers to 100 bp DNA ladder; lane 2, 4 and 6 refers to cDNA obtained from DRG of "healthy" mice and prediluted respectively 1/4, 1/16 and 1/32; lane 3, 5 and 7 refers to cDNA obtained from DRG of "asthmatic" mice and prediluted respectively 1/4, 1/16 and 1/32.
**Figure 3.** Homology between LR8 and the beta chain of the high affinity IgE receptor.

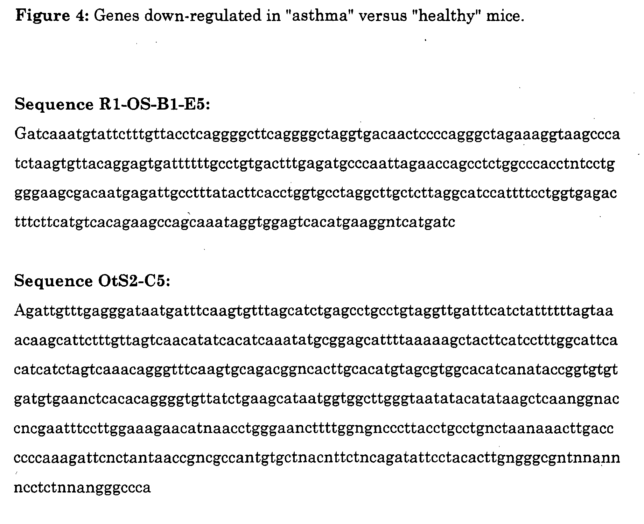

**Figure 4.** Genes down-regulated in "asthma" versus "healthy" mice.

## Claims

1. A nucleic acid library comprising genes or fragments thereof said genes essentially capable of modulating an immune response observed with airway hyperresponsiveness and/or bronchoalveolar manifestations of asthma.

2. A library according to claim 1 wherein said immune response is up-regulated.

3. A library according to claim 1 wherein said immune response is down-regulated.

4. A library according to any one of claims 1-3 wherein said library comprises nucleic acid essentially equivalent to a signature sequence as shown in table 1, 2 or 3.

5. A library according to anyone of claims 1-4 wherein at least one of said genes encodes a regulatory molecule and/or co-stimulatory molecule and/or adhesion molecule and/or receptor molecule involved in modulating an immune response.

6. A method for modulating an immune response of an individual comprising modulating a gene comprising a nucleic acid at least functionally equivalent to a nucleic acid identifiable by a signature sequence as shown in table 1, 2 or 3.

7. A method according to claim 6 wherein said gene modulates a signal transduction cascade pertaining to an immune response.

8. A method according to claim 7 wherein said signal transduction cascade modulates the production of cytokines and/or chemokines and/or growth factors.

9. A method according to anyone of claims 6-8 wherein said gene modulates sensory nerve activation.

10. A method according to anyone of claims 6 -9 wherein said gene modulates a Th1 and/or Th2 mediated immune response.

11. A method according to anyone of claims 6-10 wherein said gene modulates the generation of anti-oxidants or free radicals.

12. A method according to anyone of claims 6-11 wherein said gene modulates a CD8⁺ T-lymphocyte response.

13. A method according to claims 6-12 wherein said gene encodes a gene product capable of modulating an immune response.

14. A method according to anyone of claims 6-13 wherein said immune response comprises airway hyperresponsiveness and/or bronchoalveolar manifestations of asthma.

15. A method according to anyone of claims 6-14 wherein said gene is modulated by transducing a cell of said individual.

16. A substance capable of modulating a gene comprising a nucleic acid at least functionally equivalent to a nucleic acid identifiable by a signature sequence as shown in table 1, or 2 or 3.

17. A medicament comprising a substance according to claim 16.

18. Use of a substance according to claim 16 for the production of a medicament for the treatment of an immune response observed with airway hyperresponsiveness and/or bronchoalveolar manifestations of asthma.

19. Use of a proteinaceous substance derived from a nucleic acid at least functionally equivalent to a nucleic acid identifiable by a signature sequence as shown in table 1, 2 or 3 for the production of an antagonist against said substance.

20. Use according to claim 19 wherein said antagonist is an antibody or functional equivalent thereof.

21. An antagonist directed against a proteinaceous substance derived from a nucleic acid at least functionally equivalent to a nucleic acid identifiable by a signature sequence as shown in table 1, 2 or 3.

22. An antagonist according to claim 21 comprising an antibody or functional equivalent thereof.

23. A medicament comprising an antagonist according to claim 22.

24. Use of an antagonist according to claim 21 or 22 for the production of a medicament for the treatment of an immune response observed with airway hyperresponsiveness and/or bronchoalveolar manifestations of asthma.
